# EUROPEAN PATENT APPLICATION

(11) **EP 4 368 639 A1**
(43) Date of publication of application: **15.05.2024**
(21) Application number: 22827717.4
(22) Date of filing: 27.06.2022
(51) Int. Cl.: C07K 16/28, A61K 51/10, G01N 33/53

(54) **CD8ALPHA BINDING POLYPEPTIDE AND USE THEREOF**

(30) Priority: 23.06.2021 CN 202110697359
(71) Applicant: Suzhou Smartnuclide Biopharmaceutical Co., Ltd., Jiangsu 215100 (CN)
(72) Inventor: XU, Tao, Suzhou, Jiangsu 215123 (CN); WANG, Chao, Suzhou, Jiangsu 215123 (CN); YANG, Yanling, Suzhou, Jiangsu 215123 (CN); SUN, Yan, Suzhou, Jiangsu 215123 (CN); QI, Hao, Suzhou, Jiangsu 215123 (CN); DU, Haijing, Suzhou, Jiangsu 215123 (CN); MIAO, Liyan, Suzhou, Jiangsu 215123 (CN); WANG, Yan, Suzhou, Jiangsu 215123 (CN); QIN, Songbing, Suzhou, Jiangsu 215123 (CN)
(74) Representative: Weickmann & Weickmann PartmbB
(86) International application number: PCT/CN2022/101541
(87) International publication number: WO 2022/268225

(57) **Abstract**

Provided is a CD8α binding polypeptide. The CD8α binding polypeptide contains at least one variable domain, and compared with an amino acid sequence as represented by SEQ ID NO:1, the amino acid sequence of the variable domain has substitutions, deletions or additions of one or several amino acids. Further provided are a composition containing the CD8α binding polypeptide and the use thereof in the diagnosis and/or treatment of various diseases including, for example, tumors.

## Description

### FIELD OF THE INTVENTION

The present application relates to the field of biological medicine, and in particular, to a CD8α binding polypeptide and uses thereof.

### BACKGROUND OF THE INVENTION

Emission computed tomography (ECT) has been used in the diagnosis of tumors. ECT includes single-photon emission computed tomography (SPECT) and positron emission tomography (PET), providing high-resolution tumor imaging and image-based quantitative analysis.

In humans, CD8 is mainly expressed in cytotoxic T lymphocytes, and also in dendritic cells, natural killer cells or the like. CD8 molecules can be homodimers formed by CD8α or heterodimers formed by CD8α and CD8β, with αβ heterodimers being more prevalent.

The ability to monitor CD8-positive tumor-infiltrating lymphocytes (TILs) in vivo is of great significance for assessing responses to immunotherapies and assisting in the development of more effective single and combined therapies targeting immune cells. "ImmunoPET" development of tumor-infiltrating T cells can provide a specific and sensitive approach to help patients select a particular immunotherapeutic regimen and determine therapeutic efficacy.

There is a need in the field for a detection agent that can be used to detect CD8-expressing cells, in particular a CD8α antibody-based detection agent that is applicable to ECT detection.

### SUMMARY OF THE INVENTION

In one aspect, the present application provides a CD8α binding polypeptide, comprising at least one variable domain, wherein an amino acid sequence of the variable domain has substitution, deletion or addition of one or more amino acids compared with an amino acid sequence as set forth in SEQ ID NO.: 1.

In some embodiments, the CD8α binding polypeptide has one or more of the following properties:
(i) being capable of binding to a packing of a PROTEIN A affinity chromatographic column;
(ii) being capable of binding to CD8α with the same or greater affinity compared with a reference antibody having an amino acid sequence as set forth in SEQ ID NO.: 1;
(iii)an increased expression level compared with the reference antibody having the amino acid sequence as set forth in SEQ ID NO.: 1; and
(iv)an increased isoelectric point compared with the reference antibody having the amino acid sequence as set forth in SEQ ID NO.: 1.

In some embodiments, the CD8α binding polypeptide comprises an antibody or an antigen-binding fragment thereof.

In some embodiments, the antibody comprises a monoclonal antibody, a multispecific antibody, a chimeric antibody, a humanized antibody and/or a fully human antibody.

In some embodiments, the antigen-binding fragment comprises Fab, Fab', F(ab)₂, a Fv fragment, F(ab')₂, scFv, di-scFv, VHH and/or dAb.

In some embodiments, the VHH is camelid, chimeric, human, partially humanized or fully humanized.

In some embodiments, the variable domain comprises CDR1, CDR2 and CDR3 in an amino acid sequence as set forth in SEQ ID NO.: 14.

In some embodiments, the variable domain comprises CDR1, CDR2 and CDR3 in an amino acid sequence as set forth in SEQ ID NO.: 2, SEQ ID NO: 15 or SEQ ID NO: 16.

In some embodiments, the variable domain comprises CDR1, and the CDR1 comprises an amino acid sequence as set forth in SEQ ID NO.: 19.

In some embodiments, the variable domain comprises CDR1, and the CDR1 comprises an amino acid sequence as set forth in SEQ ID NO.: 17 or SEQ ID NO: 18.

In some embodiments, the variable domain comprises CDR2, and the CDR2 comprises an amino acid sequence as set forth in SEQ ID NO.: 23.

In some embodiments, the variable domain comprises CDR2, and the CDR2 comprises an amino acid sequence as set forth in SEQ ID NO.: 20, SEQ ID NO: 21 or SEQ ID NO: 22.

In some embodiments, the variable domain comprises CDR3, and the CDR3 comprises an amino acid sequence as set forth in SEQ ID NO.: 27.

In some embodiments, the variable domain comprises CDR3, and the CDR3 comprises an amino acid sequence as set forth in SEQ ID NO.: 24, SEQ ID NO: 25 or SEQ ID NO: 26.

In some embodiments, the variable domain comprises CDR1, CDR2 and CDR3; and the CDR1 comprises an amino acid sequence as set forth in SEQ ID NO.: 19, the CDR2 comprises an amino acid sequence as set forth in SEQ ID NO.: 23, and the CDR3 comprises an amino acid sequence as set forth in SEQ ID NO.: 27.

In some embodiments, the variable domain comprises CDR1, CDR2 and CDR3; and the CDR1 comprises an amino acid sequence as set forth in SEQ ID NO.: 17, the CDR2 comprises an amino acid sequence as set forth in SEQ ID NO.: 20, and the CDR3 comprises an amino acid sequence as set forth in SEQ ID NO.: 24.

In some embodiments, the variable domain comprises CDR1, CDR2 and CDR3; and the CDR1 comprises an amino acid sequence as set forth in SEQ ID NO.: 17, the CDR2 comprises an amino acid sequence as set forth in SEQ ID NO.: 20, and the CDR3 comprises an amino acid sequence as set forth in SEQ ID NO.: 28.

In some embodiments, the variable domain comprises CDR1, CDR2 and CDR3; and the CDR1 comprises an amino acid sequence as set forth in SEQ ID NO.: 18, the CDR2 comprises an amino acid sequence as set forth in SEQ ID NO.: 23, and the CDR3 comprises an amino acid sequence as set forth in SEQ ID NO.: 26.

In some embodiments, compared with the reference antibody having the amino acid sequence as set forth in SEQ ID NO.: 1, the variable domain of the CD8α binding polypeptide comprises amino acid substitution(s) at one or more positions selected from the group consisting of:
(a) E30; (b) S60; (c) E62; (d) S72; (e) Q78; (f) I83; (g) T85; (h) D109; and (i) M112.

In some embodiments, compared with the reference antibody having the amino acid sequence as set forth in SEQ ID NO.: 1, the variable domain of the CD8α binding polypeptide comprises one or more amino acid substitutions selected from the group consisting of:
(a) E30S, E30G, E30T or E30Y; (b) S60Y; (c) E62D; (d) S72R, S72H or S72K; (e) Q78H or Q78T; (f) I83M; (g) T85S; (h) D109A, D109V, D109L, D109I, D109G, D109S or D109T; and (i) M112L, M112A, M112V, M112I, M112G, M112S or M112L.

In some embodiments, compared with the reference antibody having the amino acid sequence as set forth in SEQ ID NO.: 1, the variable domain of the CD8α binding polypeptide comprises amino acid substitution(s) selected from one or more of the following groups:
(i) an amino acid substitution of S60Y/E62D/Q78H/I83M/T85S;
(ii) an amino acid substitution of S72R, T85S, D109A, S72R/D109A or S72R/T85S/D109A; and
(iii)an amino acid substitution of E30S/M112L.

In some embodiments, the variable domain is subject to humanized modification.

In some embodiments, compared with the reference antibody having the amino acid sequence as set forth in SEQ ID NO.: 1, the variable domain of the CD8α binding polypeptide comprises amino acid substitution(s) at one or more positions selected from the group consisting of:
(a) L2; (b) A14; (c) G56; (d) N57; (e) A75; (f) K87; (g) P88; and (h) K117.

In some embodiments, compared with the reference antibody having the amino acid sequence as set forth in SEQ ID NO.: 1, the variable domain of the CD8α binding polypeptide comprises one or more amino acid substitutions selected from the group consisting of:
(a) L2V; (b) A14P; (c) G56R; (d) N57R; (e) A75S; (f) K87R; (g) P88A; and (h) K117Q.

In some embodiments, compared with the reference antibody having the amino acid sequence as set forth in SEQ ID NO.: 1, the variable domain of the CD8α binding polypeptide comprises the following amino acid substitutions: L2Y and A14P.

In some embodiments, compared with the reference antibody having the amino acid sequence as set forth in SEQ ID NO.: 1, the variable domain of the CD8α binding polypeptide further comprises the following amino acid substitutions: A75S, K87R, P88A and/or K117Q.

In some embodiments, compared with the reference antibody having the amino acid sequence as set forth in SEQ ID NO.: 1, the variable domain of the CD8α binding polypeptide further comprises the following amino acid substitutions: G56R and/or N57R.

In some embodiments, the variable domain comprises an amino acid sequence as set forth in SEQ ID NO.: 14.

In some embodiments, the variable domain comprises an amino acid sequence as set forth in SEQ ID NO.: 2, SEQ ID NO: 15 or SEQ ID NO: 16.

In some embodiments, the variable domain comprises an amino acid sequence as set forth in SEQ ID NO.: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

In some embodiments, a C-terminus of the CD8α binding polypeptide further comprises a cysteine modification.

In some embodiments, the cysteine modification comprises the following forms: C, VDC, (GG)ₙC or (GS)ₘC, with n or m each independently selected from the group consisting of 1, 2, 3 and 4.

In some embodiments, the CD8α binding polypeptide comprises one variable domain.

In some embodiments, the CD8α binding polypeptide comprises an amino acid sequence as set forth in SEQ ID NO.: 29, SEQ ID NO: 31 or SEQ ID NO: 32.

In another aspect, the present application provides an isolated nucleic acid molecule or isolated nucleic acid molecules, encoding the CD8α binding polypeptide previously defined.

In another aspect, the present application provides a vector, comprising the nucleic acid molecule or nucleic acid molecules previously defined.

In another aspect, the present application provides a cell, comprising the nucleic acid molecule or nucleic acid molecules previously defined or the vector previously defined.

In another aspect, the present application provides a method for preparing the CD8α binding polypeptide previously defined, comprising culturing the cell previously defined under a condition allowing expression of the CD8α binding polypeptide.

In some embodiments, the method further comprises recovering a CD8α binding polypeptide expressed by the cell.

In some embodiments, the method further comprises purifying and/or modifying the CD8α binding polypeptide.

In another aspect, the present application provides an immunoconjugate, comprising the CD8α binding polypeptide previously defined.

In some embodiments, the immunoconjugate further comprises at least one detectable marker binding to the CD8α binding polypeptide.

In some embodiments, the detectable marker is selected from the group consisting of a radionuclide, a fluorescent agent, a chemiluminescent agent, a bioluminescent agent, a paramagnetic ion, and an enzyme.

In some embodiments, the detectable marker comprises ¹¹⁰In, ¹¹¹In, ¹⁷⁷Lu, ¹⁸F, ⁵²Fe, ⁶²Cu, ⁶⁷Cu, ⁶⁷Ga, ⁶⁸Ga, ⁶⁸Ge, ⁸⁶Y, ⁹⁰Y, ⁸⁹Zr, ^{94m}Tc, ¹²⁰I, ¹²³I, ¹²⁴I, ¹²⁵I, ¹³¹I, ¹⁵⁴⁻¹⁵⁸Gd, ³²P, ¹¹C, ¹³N, ¹⁵O, ¹⁸⁶Re, ¹⁸⁸Re, ⁵¹Mn, ^{52m}Mn, ⁷²As, ⁷⁵Br, ⁷⁶Br, ^{82m}Rb, ⁸³Sr or other γ-, β-, or positron-emitters.

In some embodiments, the CD8α binding polypeptide is conjugated with the detectable marker via a chelating agent.

In some embodiments, the chelating agent is selected from the group consisting of DTPA, EDTA, NOTA, DOTA, TRAP, TETA, NETA, CB-TE2A, Cyclen, Cyclam, Bispidine, TACN, ATSM, SarAr, AmBaSar, MAG3, MAG2, HYNIC, DADT, EC, NS3, H2dedpa, HBED, DFO, PEPA or HEHA, and their derivatives.

In some embodiments, the detectable marker comprises ⁶⁸Ga.

In some embodiments, the chelating agent comprises NOTA or a derivative thereof, for example, NODAGA or Maleimide-NODAGA.

In another aspect, the present application provides a composition, comprising the CD8α binding polypeptide previously defined, the nucleic acid molecule or nucleic acid molecules previously defined, the vector previously defined, the cell previously defined and/or the immunoconjugate previously defined, and optionally, a pharmaceutically acceptable carrier.

In some embodiments, the composition comprises the CD8α binding polypeptide previously defined or the immunoconjugate previously defined, wherein the composition is a detecting agent.

In some embodiments, the detecting agent is a contrast agent.

In some embodiments, the contrast agent is an ECT contrast agent.

In some embodiments, the ECT contrast agent comprises an SPECT contrast agent or a PET contrast agent.

In another aspect, the present application provides use of the CD8α binding polypeptide previously defined, the nucleic acid molecule or nucleic acid molecules previously defined, the vector previously defined, the cell previously defined, the immunoconjugate previously defined, and/or the composition previously defined in preparation of a medicament for monitoring, preventing, alleviating and/or treating tumors.

In another aspect, the present application provides use of the CD8α binding polypeptide of the present application or the immunoconjugate of the present application in preparation of a detecting agent for detecting CD8-positive cells.

In another aspect, the present application provides a method for detecting presence and/or amount of CD8 in a biological sample, comprising contacting the biological sample with the CD8α binding polypeptide previously defined, the immunoconjugate previously defined, and the composition previously defined.

In some embodiments, the contacting is conducted in vitro or ex vivo.

In some embodiments, the biological sample is a tissue.

In some embodiments, the tissue is selected from the group consisting of a blood tissue, a lymphoid tissue and a tumor tissue.

In some embodiments, the method comprises detecting presence and/or amount of CD8-positive cells in the biological sample.

In some embodiments, the presence and/or amount of the CD8-positive cells in the biological sample is determined by imaging.

In some embodiments, the presence and/or amount of the CD8-positive cells in the biological sample is determined by flow cytometry.

In some embodiments, the CD8-positive cells are CD8-positive T cells.

In another aspect, the present application provides a method for detecting and/or diagnosing CD8-related diseases or disorders, comprising administering the CD8α binding polypeptide previously defined, the immunoconjugate previously defined or the composition previously defined to a subject in need thereof.

In some embodiments, the method further comprises performing imaging on the subject.

In some embodiments, the imaging comprises ECT imaging.

In some embodiments, the ECT imaging comprises SPECT imaging or PET imaging.

In some embodiments, the CD8-related diseases or disorders comprise tumors.

In another aspect, the present application provides a method for treating tumors, comprising:
1) administering the CD8α binding polypeptide previously defined, the immunoconjugate previously defined or the composition previously defined to a subject in need thereof; and
2) determining whether a tumor in the subject comprises CD8-positive cells,
wherein if presence of the CD8-positive cells in the tumor is detected, an anti-tumor therapy is administered to the subject.

In another aspect, the present application provides a method for monitoring efficacy of an anti-tumor therapy in a subject, comprising:
1) administering the CD8α binding peptide previously defined, the immunoconjugate previously defined, or the composition previously defined to the subject suffering from a tumor and treated with the anti-tumor therapy; and
2) determining an amount of CD8-positive cells in a tumor of the subject.

In some embodiments, the presence and/or amount of the CD8-positive cells in the tumor of the subject is determined by imaging.

In some embodiments, the anti-tumor therapy is an immune checkpoint inhibitor therapy.

In some embodiments, the anti-tumor therapy is selected from administration of a PD-1 inhibitor, a PD-L1 inhibitor, a CTLA-4 inhibitor, a TIM3 inhibitor, a BTLA inhibitor, a TIGIT inhibitor, a CD47 inhibitor, a GITR inhibitor, an LAG3 inhibitor, an additional T cell co-inhibitor or a ligand antagonist, an indolamine-2,3-dioxygenase IDO inhibitor, a vascular endothelial growth factor (VEGF) antagonist, an Ang2 inhibitor, a transforming growth factor β (TGFβ) inhibitor, an epidermal growth factor receptor (EGFR) inhibitor, a CD20 inhibitor, an antibody or a vaccine for a tumor-specific antigen, an adjuvant to increase antigen presentation, a bispecific antibody, a cytotoxin, a chemoagent, cyclophosphamide, a radiotherapy, an IL-6R inhibitor, an IL-4R inhibitor, an IL-10 inhibitor, a cytokine, and an antibody-drug conjugate (ADC).

In some embodiments, the tumor comprises a solid tumor.

In some embodiments, the solid tumor is selected from the group consisting of a colorectal cancer, an ovarian cancer, a prostate cancer, a breast cancer, a brain cancer, a cervical cancer, a bladder cancer, an anal cancer, an uterine cancer, a colon cancer, a liver cancer, a pancreatic cancer, a lung cancer, an endometrial cancer, a bone cancer, a testicular cancer, a skin cancer, a renal cancer, a gastric cancer, an esophageal cancer, a head and neck cancer, a salivary gland cancer and myeloma.

In another aspect, the present application provides a method for isolating CD8-positive cells, comprising: contacting a cell population comprising CD8-positive cells with the CD8α binding polypeptide previously defined or the immunoconjugate previously defined, and recovering CD8-positive cells binding to the CD8α binding polypeptide previously defined or the immunoconjugate previously defined.

In some embodiments, the CD8-positive cells are CD8-positive T cells.

In some embodiments, the cell population comprising CD8-positive cells is human peripheral blood mononuclear cells (PBMCs).

In some embodiments, the CD8α binding polypeptide previously defined or the immunoconjugate previously defined is immobilized on a surface of a solid.

In some embodiments, the solid comprises a gel or a magnetic bead.

In another aspect, the present application provides a kit, comprising the CD8α binding polypeptide previously defined, the immunoconjugate previously defined or the composition previously defined.

Other aspects and advantages of the present application may be readily perceived by those skilled in the art from the detailed description below. The detailed description below only illustrates and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

### BRIEF DESCRIPTION OF THE DRAWINGS

The specific features of the invention involved in the present application are listed in the appended claims. The characteristics and advantages of the invention involved in the present application may be better understood by referring to the exemplary embodiments and the accompanying drawings described in detail below. The accompanying drawings are briefly illustrated as follows.
FIG. 1A shows the binding of an antibody C37 of the present application to a PROTEIN A affinity packing;
FIG. 1B shows the binding of an antibody C37-YDHMS of the present application to the PROTEIN A affinity packing;
FIG. 2 shows an SEC-HPLC purity analysis chart of an antibody GSC-C37H of the present application before and after conjugation and after reduction;
FIG. 3 shows the mass spectrometry data of the antibody GSC-C37H of the present application before and after conjugation with NODAGA;
FIG. 4 shows the ELISA data of the antibody GSC-C37H of the present application before and after conjugation with NODAGA;
FIG. 5A shows the Radio-TLC results of a ⁶⁸Ga-NODAGA-GSC-C37H single-domain antibody of the present application;
FIG. 5B shows the Radio- HPLC results of the ⁶⁸Ga-NODAGA-GSC-C37H single-domain antibody of the present application;
FIG. 6 shows the PET/CT imaging of the ⁶⁸Ga-NODAGA-GSC-C37H of the present application in an HSC-NPG animal model;
FIG. 7 shows the micro-PET imaging of the ⁶⁸Ga-NODAGA-GSC-C37H of the present application in a PBMC animal model;
FIG. 8A shows the biodistribution results (1 h after administration) of ROI after micro-PET development in PBMC humanized animals according to the present application;
FIG. 8B shows the biodistribution results (2 h after administration) of ROI after 2 hours since micro-PET development in BMC humanized animals according to the present application;
FIG. 8C shows the ex vivo biodistribution results (2 h after administration) of dissection after 2 hours since administration to PBMC humanized animals according to the present application;
FIG. 9A shows the trend of blood radioactivity ID%/g over time of PBMC humanized animals according to the present application;
FIG. 9B shows the %ID/g TAC curves for radioactive substance uptake in different doses of blood of PBMC humanized animals according to the present application;
FIG. 10A shows the Radio-TLC assay results of the in vitro serum stability of the ⁶⁸Ga-NODAGA-GSC-C37H single-domain antibody of the present application;

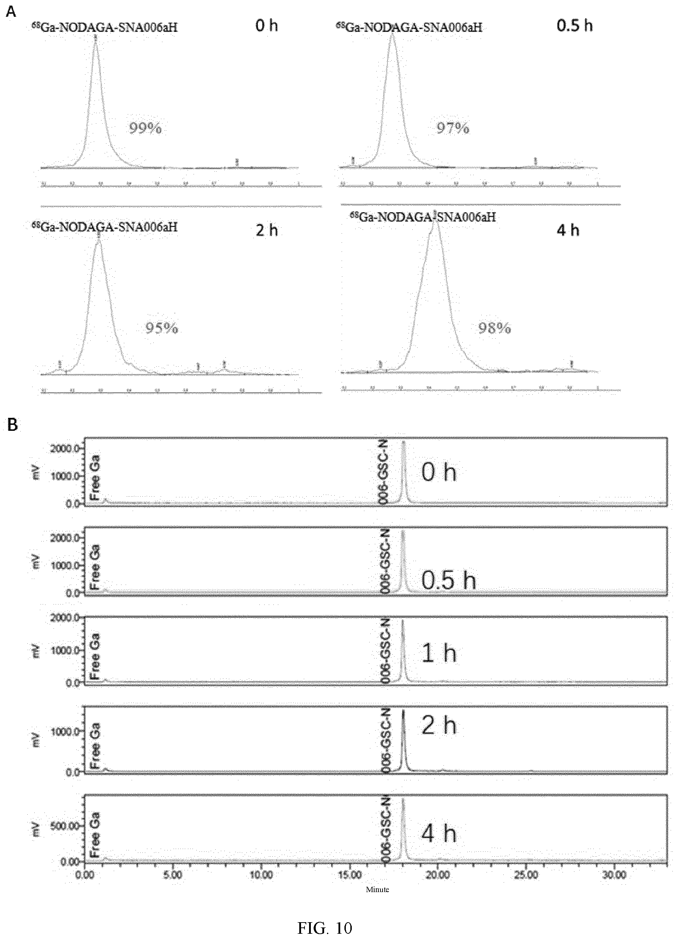

FIG. 10A shows the Radio- HPLC assay results of the in vitro serum stability of the ⁶⁸Ga-NODAGA-GSC-C37H single-domain antibody of the present application;

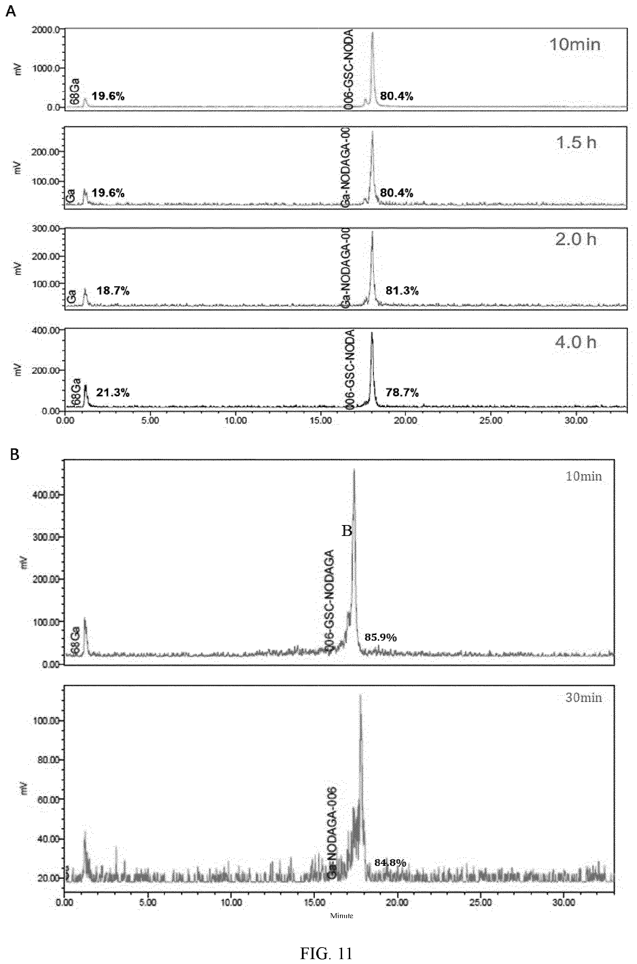

FIG. 11A shows the Radio-HPLC results of urine samples from mice at different times after dosing according to the present application;
FIG. 11B shows the Radio-HPLC results of blood samples from mice at different times after dosing according to the present application; and

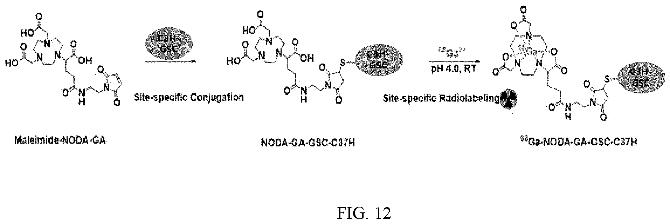

FIG. 12 shows a synthesis roadmap of the ⁶⁸Ga-NODA-GA-GSC-C37H of the present application.

### DETAILED DESCRIPTION OF THE PREFERRED EMBODIMENTS

The embodiments of the invention of the present application will be illustrated by specific examples below. Those familiar with this technology may easily understand other advantages and effects of the invention of the present application from the content disclosed in the specification.

### TERMS & DEFINITIONS

The term "CD8" (cluster of differentiation 8) refers to cell surface glycoproteins expressed primarily on cytotoxic T lymphocytes, and also on subsets of dendritic cells, natural killer cells, natural killer T cells, and γ δ T cells. The glycoproteins are composed of two isoforms α and β (which are encoded by different genes) and are expressed as αα homodimers or αβ heterodimers, with the latter predominant. A CD8 coreceptor stabilizes the interaction between a T cell receptor and MHC-1, and initiates intracellular signaling through phosphorylation of a lymphocyte-specific protein tyrosine kinase (Lck) of a CD3-associated immunoreceptor tyrosine-based activation motif (ITAM) to facilitate activation.

In the present application, the term "CD8α binding polypeptide" means any polypeptide capable of specifically binding to CD8α. In some embodiments, the binding polypeptide is an antibody. In other embodiments, the binding polypeptide is, for example, an antibody mimic, a cytokine, or a growth factor, for example, a single-domain antibody specifically binding to CD8α in the present application. Or, the "CD8α binding polypeptide" may refer to a monovalent polypeptide binding to CD8α (i.e., a polypeptide binding to one epitope of CD8α), and a bivalent or polyvalent binding polypeptide (i.e., a binding polypeptide binding to more than one epitope). The "CD8α binding polypeptide" in the present application may comprise at least one variable domain binding to CD8α. In some embodiments, the "CD8α binding polypeptide" of the present application may comprise 2, 3, 4, or more variable domains binding to CD8α. In addition to the variable domain binding to CD8α, the CD8α binding polypeptide of the present application may also comprise a linker and/or a moiety having an effector function, for example, a half-life extension moiety (for example, a variable domain binding to serum albumin), and/or a fusion partner (for example, serum albumin) and/or a conjugated polymer (for example, PEG) and/or a Fc region. In some embodiments, the "CD8α binding polypeptide" of the present application also covers a bispecific antibody, which comprises a variable domain binding to different antigens.

In the present application, the term "antibody" is generally used in the broadest sense and specifically covers monoclonal antibodies, polyclonal antibodies, dimers, multimers, polyspecific antibodies (for example, bispecific antibodies), and antibody fragments, provided that they show the desirable biological activity (Milleretal (2003), Jour. Of Immunology, 170: 4854-4861), i.e., the capability of binding to CD8α (such as human CD8α, mouse CD8α, cynomolgus monkey CD8α or rhesus monkey CD8α. The antibody may be a mouse, human, humanized, or chimeric antibody, or may be derived from other species.

A full-length antibody typically refers to an antibody consisting of two "full-length antibody heavy chains" and two "full-length antibody light chains". The "full-length antibody heavy chain" is generally a polypeptide formed by an antibody heavy-chain variable domain (VH), an antibody heavy-chain constant domain 1 (CH1), an antibody hinge region (HR), an antibody heavy-chain constant domain 2 (CH2), and an antibody heavy-chain constant domain 3 (CH3) in a direction from an N-terminus to a C-terminus, abbreviated as VH-CH1-HR-CH2-CH3. Moreover, in the case of antibodies of an IgE subclass, an antibody heavy-chain constant domain 4 (CH4) is optionally included. In some embodiments, the "full-length antibody heavy chain" is a polypeptide formed by VH, CH1, HR, CH2, and CH3 in the direction from the N-terminus to the C-terminus. The "full-length antibody light chain" is generally a polypeptide formed by an antibody light-chain variable domain (VL) and an antibody light-chain constant domain (CL) in the direction from the N-terminus to the C-terminus, abbreviated as VL-CL. The antibody light-chain constant domain (CL) may be either κ (kappa) or λ (lambda). The two full-length antibody chains are linked together by an inter-polypeptide disulfide bond between the CL domain and the CH1 domain and an inter-polypeptide disulfide bond between the hinge regions of the full-length antibody heavy chains. Typical examples of the full-length antibody are natural antibodies such as IgG (for example, IgG1 and IgG2), IgM, IgA, IgD, and IgE).

In the present application, the term "antigen-binding fragment" generally refers to a portion of an antibody molecule that contains amino acids responsible for the specific binding between an antibody and an antigen. The portion of the antigen that is specifically recognized and bound by the antibody is called the "epitope" as described above. The antigen-binding domain may typically comprise an antibody light-chain variable region (VL) and an antibody heavy-chain variable region (VH); however, it is unnecessary to comprise both. A Fd fragment has, for example, two VH regions and generally retains some of the antigen binding functions of the complete antigen-binding domain. Examples of antigen-binding fragments of antibodies comprise: (1) Fab fragments, which are monovalent fragments having VL, VH, constant light chains (CL) and CH1 domains; (2) F(ab')2 fragments, which are bivalent fragments in which two Fab fragments are linked by a disulfide bridge in a hinge region; (3) Fd fragments having two VH and CH1 domains; (4) Fv fragments having VL and VH domains of single antibody arms, (5) dAb fragments (Ward et al., "Binding Activities of a Repertoire of Single Immunoglobulin Variable Domains Secreted From Escherichia coli," Nature, 341:544-546(1989), which is incorporated herein by reference in its entirety) having VH domains; (6) isolated complementarity-determining regions (CDRs), and (7) single-chain Fvs (scFvs), which are, for example, derived from a scFV-library. Although the two domains VL and VH of the Fv fragments are encoded by independent genes, they can be joined using a recombinant method by synthetic linkers, which allow them to be prepared as single proteins in which the VL and VH regions are paired to form monovalent molecules (called single-chain Fvs (scFvs)) (see, for example, Huston et al., "Protein Engineering of Antibody Binding Sites: Recovery of Specific Activity in an Anti-Digoxin Single-Chain Fv Analogue Produced in Escherichia coli," Proc. Natl. Acad. Sci. USA 85:5879-5883(1988)); and (8) VHH, where "VHH" involves antigen-binding variable domains of heavy-chain antibodies from the family Camelidae (camel, dromedary, llama, alpaca or the like) (see Nguyen V K. et. al., 2000, The EMBO Journal, 19, 921-930; Muyldermans S., 2001, J Biotechnol., 74, 277-302 and the review Vanlandschoot P. et al., 2011, Antiviral Research, 92, 389-407). VHH may also be referred to as nanobody (Nb) and/or single-domain antibody. These antibody fragments are obtained using conventional techniques known to those skilled in the art, and their functions are evaluated in the same way as that for the complete antibodies.

In the present application, the term "variable domain" generally refers to an antibody variable domain capable of specifically binding to an epitope, for example, antibody variable domains VH and VL (VH domain and VL domain). Another example of the variable domain is the "VHH domain" (or "VHH" for short). The "VHH domain", also known as heavy-chain single-domain antibody, VHH, VHH domain, VHH antibody fragment, and VHH antibody, is a variable domain of an antigen-binding immunoglobulin called "heavy chain antibody" (i.e., "antibody lacking light chains") (Hamers-Casterman C, Atarhouch T, Muyldermans S, Robinson G, Hamers C, Songa EB, Bendahman N, Hamers R.: "Naturally occurring antibodies devoid of light chains"; Nature 363,446-448(1993)). The term "VHH domain" is used to distinguish the variable domain described from the heavy-chain variable domain (which is referred to as the "VH domain" in the present application) present in a conventional 4-chain antibody and the light-chain variable domain (which is referred to as the "VL domain" in the present application) present in a conventional 4-chain antibody. The VHH domain specifically binds to an epitope without the need for other antigen-binding domains (this is opposite to the VH or VL domain in a conventional 4-chain antibody, where the epitope is recognized by the VL domain along with the VH domain).

In the present application, the "variable domains" generally have the same general structure, and each domain comprises four framework regions (FRs) with highly conserved sequences, where the FR comprises four "framework regions", namely, "framework region 1" or "FR1", "framework region 2" or "FR2", "framework region 3" or "FR3", and "framework region 4" or "FR4", and the "complementarity-determining region 1" or "CDR1", the "complementarity-determining region 2" or "CDR2", and the "complementarity-determining region 3" or "CDR3" in the FR are linked. The general structure or sequence of the variable domain can be expressed as follows: FR1-CDR1-FR2-CDR2-FR3-CDR3-FR4. The variable domain of an antibody has an antigen-binding site, thereby endowing the antibody with specificity to an antigen.

In the present application, the term "CDR" generally refers to a complementarity-determining region in a variable sequence of an antibody. Each variable region of the heavy and light chains comprises 3 CDRs, called CDR1, CDR2, and CDR3 for each variable region. The precise boundaries of these CDRs have been defined differently depending on the system. The system described by Kabat (Kabat et al., Sequences of Proteins of Immunological Interest, National Institutes of Health, Bethesda, Md. (1987) and (1991)) provides a definite residue numbering system applicable to any variable region of an antibody, aand also provides precise residue boundaries for defining the three CDRs. These CDRs may be referred to as Kabat CDRs. Chothia and her colleagues (Chothia & Lesk, J.MoI.Biol.196:901-917(1987) and Chothia et al., Nature 342:877-883(1989)) found that some subfractions within the Kabat CDRs took almost identical peptide backbone conformation, although with great differences at the amino acid sequence level. These subsections are designated as L1, L2, and L3 or H1, H2, and H3, with "L" and "H" referring to light and heavy chain regions, respectively. These regions can be called Chothia CDRs, which have boundaries that overlap with those of the Kabat CDRs. Padlan (FASEB J.9:133-139(1995)) and MacCallum (JMoI Biol 262(5):732-45(1996)) have described additional boundaries that define CDRs overlapping with Kabat CDRs. These additional CDR boundaries may not strictly follow one of the above systems, but still overlap with Kabat CDRs, although they can be shortened or lengthened based on the following predictions or experimental findings, without significant effect on antigen binding at specific residues or residue groups or even the entire CDRs. Unless otherwise expressly stated in the specification, the terms "CDR", "HCDR1", "HCDR2", "HCDR3", "LCDR1", "LCDR2" and "LCDR3", as used in the present application, include CDRs as defined by any of the methods described above (Kabat, Chothia or IMGT).

In the present application, the term "sequence identity" usually refers to the same nucleic acid or amino acid sequences between two or more aligned sequences when sequence alignment procedures are used for alignment. The term "% sequence identity" here generally refers to the level of identity of the nucleic acid or amino acid sequences between two or more aligned sequences when sequence alignment procedures are used for alignment. Methods used to evaluate the degree of sequence identity between amino acids or nucleotides are known to those skilled in the art. For example, the identity of amino acid sequences is generally measured using sequence analysis software. For example, the BLAST program of the NCBI database may be used to determine the identity. To determine the sequence identity, see for example, Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G.,eds., Humana Press, New Jersey, 1994; Sequence Analysis in Molecular Biology, 20 von Heinje, G., Academic Press, 1987 and Sequence Analysis Primer, Gribskov, M. and Devereux, J., eds., M Stockton Press, New York, 1991.

In the present application, the amino acid residues will be represented according to the standard three- or one-letter amino acid coding that is well known and agreed upon in the art. When two amino acid sequences are aligned, the term "amino acid difference" generally refers to the insertion, deletion, or substitution of a specified number of amino acid residues at a certain position in a reference sequence as compared to the other sequence. In some embodiments, the substitution is conserved amino acid substitution, which refers to the replacement of an amino acid residue by an additional amino acid residue having a similar chemical structure, and the conserved amino acid substitution has a small or substantially no effect on the function, activity or other biological properties of the polypeptide. The conserved amino acid substitution is well known in this art. For example, the conserved amino acid substitution is the substitution of one amino acid in the following groups (i)-(v) by another amino acid residue in the same group: (i) smaller aliphatic non-polar or weakly-polar residues: Ala, Ser, Thr, Pro and Gly; (ii) negatively-charged polar residues and their (uncharged) amides: Asp, Asn, Glu and Gln; (iii) positively-charged polar residues: His, Arg and Lys; (iv) larger aliphatic non-polar residues: Met, Leu, Ile, Val and Cys; and (v) aromatic residues: Phe, Tyr and Trp. Particularly preferred conserved amino acid substitutions are as follows: substitution of Ala by Gly or Ser; substitution of Arg by Lys; substitution of Asn by Gln or His; substitution of Asp by Glu; substitution of Cys by Ser; substitution of Gln by Asn; substitution of Glu by Asp; substitution of Gly by Ala or Pro; substitution of His by Asn or Gln; substitution of Ile by Leu or Val; substitution of Leu by Ile or Val; substitution of Lys by Arg, Gln or Glu; substitution of Met by Leu, Tyr or Ile; substitution of Phe by Met, Leu or Tyr; substitution of Ser by Thr; substitution of Thr by Ser; substitution of Trp by Tyr; substitution of Tyr by Trp or Phe; and substitution of Val by Ile or Leu. In some embodiments, the substitution is a non-conserved amino acid substitution, for example, substitution of Ala by Asp, Asn, Glu, or Gin.

In the present application, the term "affinity" generally refers to the total strength of non-covalent interactions between a single binding site of a molecule (for example, a polypeptide or antibody) and its binding partner (for example, a target or antigen). The affinity of a molecule X to its partner Y can generally be expressed by a dissociation constant (Kd). Affinity may be measured by common methods (such as surface plasmon resonance) known in the art, and those methods reported in the present application are also included. The higher affinity of the molecule X to its binding partner Y can be seen in a lower Kd value and/or EC50 value.

In the present application, the term "isolated" generally refers to the at least partial isolation of a molecule (for example, an antibody, nucleic acid or the like) from other molecules to which the molecule normally binds in its native state. An "isolated polypeptide" is substantially free of other biomolecules, such as nucleic acids, proteins, lipids, carbohydrates, cell debris, and growth media. An "isolated nucleic acid" is generally present in a form or background different from its naturally occurring form.

In the present application, the term "immunoconjugate" generally refers to a conjugate formed by the linkage of an antibody or its antibody fragment to an additional active agent, such as a chemotherapeutic agent, a toxin, an immunotherapeutic agent, a radioactive element, an imaging probe, a spectral probe, or the like. The linkage may be a covalent bond, or is conducted by means of a non-covalent interaction via electrostatic force, for example. A variety of linkers known in the art may be used to form the immunoconjugate. The conjugate may specifically bind to an antigen on a target cell by the antibody or its antigen-binding fragment to deliver the additional agent to the target cell (for example, a tumor cell). In addition, this immunoconjugate may be provided as a fusion protein that can be expressed from a polynucleotide encoding the immunoconjugate.

In the present application, the term "chelating agent" generally refers to an organic molecule capable of forming a complex with a metal ion. The chelating agent is often used to label a protein or peptide. The end product of a metal ion conjugate is for use in radioimmunoassay, radioimmunotherapy, magnetic resonance imaging, photodynamic therapy, or other similar modes. Non-limiting examples of the chelating agent or a complexing agent include DTPA (diethylenetriamine pentaacetic anhydride) and derivatives thereof, NOTA (1,4,7-triazacyclononane-N,N',N"-triacetic acid) and derivatives thereof (such as NODA-GA (NODAGA), and Maleimide-NODAGA), DOTA (1,4,7,10-tetraazacyclododecane-N,N',N",N‴-tetraacetic acid) (binding to radioactive metal ions) and derivatives thereof, TETA ( 1,4,8,11-tetraazacyclotetradecane-N,N',N",N‴-tetraacetic acid) and derivatives thereof, and DTTA (N-(p-benzyl-isothiocyanate)-diethylenetriamine-N,N',N",N"-tetraacetic acid). These and other chelating agents are readily available from commercial sources.

In the present application, the term "pharmaceutically acceptable carrier" generally refers to one or more non-toxic materials that do not interfere with the effectiveness of the biological activity of an active ingredient. Such formulations may routinely comprise salts, buffers, preservatives, compatible carriers, and optionally additional therapeutic agents. Such pharmaceutically acceptable formulations may also comprise compatible solid or liquid fillers, diluents, or encapsulating substances suitable for administration to humans. Other contemplated carriers, excipients, and/or additives that may be used in the formulations described here include, for example, flavorings, antimicrobials, sweeteners, antioxidants, antistatic agents, lipids, protein excipients (such as serum albumin, gelatin, casein), salt-forming equilibrium ions (such as sodium) or the like. These and other known pharmaceutical carriers, excipients, and/or additives suitable for use in the formulations described here are known in the art, as listed in for example "Remington: The Science & Practice of Pharmacy," 21st Edition, Lippincott Williams & Wilkins (2005) and "Physician's Desk Reference," 60th Edition, Medical Economics, Montvale, New Jersey (2005). A pharmaceutically acceptable carrier that is appropriate for the desired or required means of administration, solubility, and/or stability can be routinely selected.

In the present application, the term "administer" and similar terms are generally not limited to physical administration, and appropriate methods include an in vitro method, an in vitro and then in vivo method or an in vivo method. For example, any administration method known to a person skilled in the art for contacting a cell, an organ or a tissue with the combination may be used, For example, the compound may be introduced into the body of a subject in need of treatment by any route of introduction or delivery. In some embodiments, the composition of the present application may be administered orally, topically, intranasally, intramuscularly, subcutaneously, intradermally, intrathecally, intraperitoneally, or percutaneously.

In the present application, the terms "ex vivo" and "in vitro" are interchangeable, and generally refer to the conduction of activities in cells, tissues, and/or organs that have been removed from the body of a subject in a controlled environment.

In the present application, the term "diagnosis" generally refers to the detection of a disease or disorder, or the determination of the state or extent of a disease or disorder. The term "diagnosis" may also include detection of a trigger for a disease or disorder, determination of the therapeutic effect of a drug therapy, or prediction of a response pattern to a drug therapy.

In the present application, the term "treatment" generally refers to: (i) prevention of the development of a disease, disorder or condition in a patient who may be susceptible to this disease, disorder and/or condition, but has not yet been diagnosed with this disease, disorder and/or condition; (ii) inhibition of this disease, disorder or condition, i.e. containment of its development; and (iii) alleviation of this disease, disorder and/or condition, such that this disease, disorder and/or condition and/or symptoms associated with this disease, disorder and/or condition disappear.

In the present application, the terms "tumor" and "cancer" are used interchangeably and generally refer to neoplastic or malignant cell growth. The tumor in the present application may be benign or malignant. The tumor in the present application may be solid or non-solid.

As used herein, the term "subject" generally refers to a human or non-human animal, comprising but not limited to a cat, a dog, a horse, a pig, a cow, a goat, a rabbit, a mouse, a rat, or a monkey.

In the present application, the term "about" generally refers to a variation within a range of 0.5%-10% above or below a specified value, for example, a variation within a range of about 0.5%, about 1%, about 1.5%, about 2%, about 2.5%, about 3%, about 3.5%, about 4%, about 4.5%, about 5%, about 5.5%, about 6%, about 6.5%, about 7%, about 7.5%, about 8%, about 8.5%, about 9%, about 9.5%, or about 10% above or below a specified value.

In the present application, the term "comprise" and its variant forms, including "comprises", "comprising" and other forms, generally refer to the inclusion of other components, elements, numerical values, steps or the like.

### DETAILED DESCRIPTION OF THE INVENTION

### CD8α Binding Polypeptide

In one aspect, the present application provides a CD8α binding polypeptide. The CD8α binding polypeptide may comprise at least one variable domain, wherein an amino acid sequence of the variable domain has substitution, deletion or addition of one or more amino acids compared with an amino acid sequence as set forth in SEQ ID NO.: 1.

In some embodiments, the CD8α binding polypeptide may comprise one, or two, or three variable domains.

In some embodiments, with respect to SEQ ID NO.: 1, the variable domain may comprise an amino acid sequence having substitution, deletion or addition of one, or two, or three, or four, or five, or six, or seven, or eight, or nine, or ten, or eleven, or twelve, or thirteen, or fourteen, or fifteen, or sixteen, or seventeen, or eighteen, or nineteen, or twenty amino acids.

In some embodiments, an amino acid sequence of the variable domain may have substitution of one or more amino acids, and may comprise conserved and/or non-conserved substitution compared with the amino acid sequence as set forth in SEQ ID NO.: 1.

In some embodiments, an amino acid mutation may occur in the CDR (for example, CDR1, CDR2, or CDR3) of a targeted fraction. In another embodiment, an amino acid change may occur in the framework region (FR) (for example, FR1, FR2, FR3, or FR4) of a targeted fraction.

The modification of an amino acid sequence may be achieved by using any known technique in the art, for example, site-directed mutagenesis or PCR-based mutagenesis. Such techniques are described in the following literature, for example: Sambrook et al., Molecular Cloning: A Laboratory Manual, Cold Spring Harbor Press, Plainview, N.Y,1989; and Ausubel et al., Current Protocols in Molecular Biology, John Wiley&Sons, New York, N.Y,1989.

In some embodiments, the substitution, deletion or addition substantially does not reduce the ability of the CD8α binding polypeptide of the present application to specifically bind to CD8α.

The equilibrium dissociation constant (KD) may be used to describe the binding affinity of the CD8α binding polypeptide of the present application to the full-length and/or mature form of human CD8α and/or its isoform and/or splice variant and/or fragment and/or any other naturally occurring or synthetic analogue, variant, or mutant (including monomer, dimer, heterodimer, multimer and/or related forms). The CD8α binding polypeptide may comprise a targeting moiety that binds to the full-length and/or mature form of human CD8α and/or its isoform and/or splice variant and/or fragment and/or any other naturally occurring or synthetic analogue, variant, or mutant (including monomer, dimer, heterodimer, multimer and/or related forms), with KD lower than about 1 uM, about 900 nM, about 800 nM, about 700 nM, about 600 nM, about 500 nM, about 400 nM, about 300 nM, about 200 nM, about 100 nM, about 90 nM, about 80 nM, about 70 nM, about 60 nM, about 50 nM, about 40 nM, about 30 nM, about 20nM, about 10 nM, or about 5 nM, or about 1 nM.

In some embodiments, the CD8α binding polypeptide may have one or more of the following properties:
(i) being capable of binding to a packing of a PROTEIN A affinity chromatographic column;
(ii) being capable of binding to CD8α with the same or greater affinity compared with a reference antibody having an amino acid sequence as set forth in SEQ ID NO.: 1;
(iii)an increased expression level compared with the reference antibody having the amino acid sequence as set forth in SEQ ID NO.: 1; and
(iv)an increased isoelectric point compared with the reference antibody having the amino acid sequence as set forth in SEQ ID NO.: 1.

In some embodiments, compared with the reference antibody having the amino acid sequence as set forth in SEQ ID NO.: 1, the CD8α binding polypeptide of the present application has an expression of about 5%, 10%, 20%, 30%, 40%, 50 %, 60 %, 70 %, 80 %, 90 % or higher.

In some embodiments, the reference antibody having the amino acid sequence as set forth in SEQ ID NO.: 1 has an isoelectric point (PI) value of 4.56, and the CD8α binding polypeptide has a PI value of not less than 4.56. For example, the PI value of the CD8α binding polypeptide may be about 5, about 5.5, about 6.0, about 6.5, about 6.6, about 6.7, about 6.8, about 6.9, or about 7.

In some embodiments, the CD8α binding polypeptide comprises an antibody or an antigen-binding fragment thereof.

In some embodiments, the antibody may comprise a monoclonal antibody, a multispecific antibody (for example, a bispecific antibody), a chimeric antibody, a humanized antibody and/or a fully human antibody.

In some embodiments, the antigen-binding fragment may comprise Fab, Fab', F(ab)₂, a Fv fragment, F(ab')₂, scFv, di-scFv, VHH and/or dAb. For example, the antibody or the antigen-binding fragment thereof may be VHH.

In some embodiments, the VHH is not limited to a specific biological original or a specific preparation method. For instance, the VHH may generally be obtained by: (1) isolation of the VHH domain of a naturally occurring heavy chain antibody; (2) expression of a nucleotide sequence encoding the naturally occurring VHH domain; (3) "humanization" of the naturally occurring VHH domain or expression of a nucleic acid encoding such a humanized VHH domain; (4) "camelization" of a naturally occurring VH domain from any animal species, such as mammalian species, such as humans, or expression of a nucleic acid encoding such a camelized VH domain; (5) "camelization" of a "domain antibody" or "Dab" as described in the art, or expression of a nucleic acid encoding such a camelized VH domain; (6) preparation of a known protein, a polypeptide or other amino acid sequences in the art by using a synthetic or semi-synthetic technique; (7) preparation of a nucleic acid encoding VHH by using a nucleic acid synthesis technique known in the art, and subsequent expression of a nucleic acid obtained in such a way; and/or (8) any combination of one or more of the above.

In some embodiments, the CD8α binding polypeptide may comprise VHH that has been "camelized", i.e., by replacing one or more amino acid residues in an amino acid sequence of a naturally occurring VH domain of a conventional 4-chain antibody by one or more amino acid residues present at the corresponding position(s) in the VHH domain of a camel heavy chain antibody. In some embodiments, such "camelization" substitution is inserted into an amino acid position formed and/or present at a VH-VL interface and/or into a so-called camelidae labeled residue (see, for example, WO9404678, the entire content of which is incorporated into the present application by reference). In some embodiments, a VH sequence used as a starting substance or starting point for the production or design of a camelized VHH is a VH sequence from a mammal, for example, a human VH sequence, such as a VH3 sequence. The camelized VHH may be obtained by any suitable means known in the art (i.e., as indicated in points (1) to (8) above), and is thus not strictly limited to a polypeptide that is already obtained by using a polypeptide comprising a naturally occurring VH domain as a starting substance.

In some embodiments, the VHH may be camelid, chimeric, human, partially humanized or fully humanized.

In some embodiments, the CD8α binding polypeptide may comprise VHH that has been "humanized", i.e., by replacing one or more amino acid residues in an amino acid sequence (and specifically in a framework sequence) of a naturally occurring VH sequence by one or more amino acid residues present at the corresponding position(s) in the VH domain of a human conventional 4-chain antibody. This can be done using humanized techniques known in the art. In some embodiments, possible humanized substitutions or combinations of humanized substitutions may be determined by methods known in the art, for example, by comparing a VHH sequence with a sequence of a naturally occurring human VH domain. In some embodiments, the humanized substitution is selected such that the resulting humanized VHH retains favorable functional properties. In general, as a result of humanization, the VHH of the present application may become more "human-like" than the corresponding naturally-occurring VHH domain, while still retaining favorable properties such as reduced immunogenicity. The humanized VHH of the present application may be obtained by any suitable means known in the art, and is thus not strictly limited to a polypeptide that is already obtained using a polypeptide comprising a naturally occurring VHH domain as a starting substance.

In some embodiments, the variable domain may comprise CDR1, CDR2 and CDR3 in an amino acid sequence as set forth in SEQ ID NO.: 14. The CDR may be Kabat CDR, AbM CDR, Chothia CDR or Contact CDR. In some embodiments, the CDR is Chothia CDR.

In some embodiments, the variable domain may comprise CDR1, CDR2 and CDR3 in an amino acid sequence as set forth in SEQ ID NO.: 2, SEQ ID NO: 15 or SEQ ID NO: 16.

In some embodiments, the variable domain may comprise CDR1, which may comprise an amino acid sequence as set forth in SEQ ID NO.: 19.

In some embodiments, the variable domain may comprise CDR1, which may comprise an amino acid sequence as set forth in SEQ ID NO.: 17 or SEQ ID NO: 18.

In some embodiments, the variable domain may comprise CDR2, which may comprise an amino acid sequence as set forth in SEQ ID NO.: 23.

In some embodiments, the variable domain may comprise CDR2, which may comprise an amino acid sequence as set forth in SEQ ID NO.: 20, SEQ ID NO: 21 or SEQ ID NO: 22.

In some embodiments, the variable domain may comprise CDR3, which may comprise an amino acid sequence as set forth in SEQ ID NO.: 27.

In some embodiments, the variable domain may comprise CDR3, which may comprise an amino acid sequence as set forth in SEQ ID NO.: 24, SEQ ID NO: 25 or SEQ ID NO: 26.

For example, the variable domain may comprise CDR1, CDR2 and CDR3; and the CDR1 may comprise an amino acid sequence as set forth in SEQ ID NO.: 19, the CDR2 may comprise an amino acid sequence as set forth in SEQ ID NO.: 23, and the CDR3 may comprise an amino acid sequence as set forth in SEQ ID NO.: 27.

For example, the variable domain may comprise CDR1, CDR2 and CDR3; and the CDR1 may comprise an amino acid sequence as set forth in SEQ ID NO.: 17, the CDR2 may comprise an amino acid sequence as set forth in SEQ ID NO.: 20, and the CDR3 may comprise an amino acid sequence as set forth in SEQ ID NO.: 24.

For example, the variable domain may comprise CDR1, CDR2 and CDR3; and the CDR1 may comprise an amino acid sequence as set forth in SEQ ID NO.: 17, the CDR2 may comprise an amino acid sequence as set forth in SEQ ID NO.: 20, and the CDR3 may comprise an amino acid sequence as set forth in SEQ ID NO.: 28.

For example, the variable domain may comprise CDR1, CDR2 and CDR3; and the CDR1 may comprise an amino acid sequence as set forth in SEQ ID NO.: 18, the CDR2 may comprise an amino acid sequence as set forth in SEQ ID NO.: 23, and the CDR3 may comprise an amino acid sequence as set forth in SEQ ID NO.: 26.

In some embodiments, compared with the reference antibody having the amino acid sequence as set forth in SEQ ID NO.: 1, the variable domain of the CD8α binding polypeptide may comprise amino acid substitution(s) at one or more positions selected from the group consisting of:
(a) E30; (b) S60; (c) E62; (d) S72; (e) Q78; (f) I83; (g) T85; (h) D109; and (i) M112.

For example, compared with the reference antibody having the amino acid sequence as set forth in SEQ ID NO.: 1, the variable domain of the CD8α binding polypeptide may comprise amino acid substitutions at the following positions: (b) S60; (c) E62; (e) Q78; (f)I83; and (g) T85.

For example, compared with the reference antibody having the amino acid sequence as set forth in SEQ ID NO.: 1, the variable domain of the CD8α binding polypeptide may comprise amino acid substitutions at the following positions: (d)S72; (f)I83; and/or (h) D109.

For example, compared with the reference antibody having the amino acid sequence as set forth in SEQ ID NO.: 1, the variable domain of the CD8α binding polypeptide may comprise amino acid substitutions at the following positions: (a) E30; and/or (i) M112.

For example, compared with the reference antibody having the amino acid sequence as set forth in SEQ ID NO.: 1, the variable domain of the CD8α binding polypeptide may comprise amino acid substitutions at the following positions: (a) E30; (b) S60; (c) E62; (d) S72; (e) Q78; (f) I83; (g) T85; (h) D109; and (i) M112.

In some embodiments, compared with the reference antibody having the amino acid sequence as set forth in SEQ ID NO.: 1, the variable domain of the CD8α binding polypeptide may comprise one or more amino acid substitutions selected from the group consisting of:
(a) E30S, E30G, E30T or E30Y; (b) S60Y; (c) E62D; (d) S72R, S72H or S72K; (e) Q78H or Q78T; (f) I83M; (g) T85S; (h) D109A, D109V, D109L, D109I, D109G, D109S or D109T; and (i) M112L, M112A, M112V, M112I, M112G, M112S or M112L.

In some embodiments, compared with the reference antibody having the amino acid sequence as set forth in SEQ ID NO.: 1, the variable domain of the CD8α binding polypeptide may comprise one or more amino acid substitutions selected from the group consisting of:
(a) E30S; (b) S60Y; (c) E62D; (d) S72R; (e) Q78H; (f) I83M; (g) T85S; (h) D109A; and (i) M112L.

In some embodiments, compared with the reference antibody having the amino acid sequence as set forth in SEQ ID NO.: 1, the variable domain of the CD8α binding polypeptide may comprise amino acid substitution(s) selected from one or more of the following groups:
(i) an amino acid substitution of S60Y/E62D/Q78H/I83M/T85S;
(ii) an amino acid substitution of S72R, T85S, D109A, S72R/D109A or S72R/T85S/D109A; and
(iii)an amino acid substitution of E30S/M112L.

For example, compared with the reference antibody having the amino acid sequence as set forth in SEQ ID NO.: 1, the variable domain of the CD8α binding polypeptide may comprise the following amino acid substitutions: S60Y, E62D, Q78H, I83M and T85S.

For example, compared with the reference antibody having the amino acid sequence as set forth in SEQ ID NO.: 1, the variable domain of the CD8α binding polypeptide may comprise the following amino acid substitutions: S72R, T85S, and D109A.

For example, compared with the reference antibody having the amino acid sequence as set forth in SEQ ID NO.: 1, the variable domain of the CD8α binding polypeptide may comprise the following amino acid substitutions: E30S and M112L.

For another example, compared with the reference antibody having the amino acid sequence as set forth in SEQ ID NO.: 1, the variable domain of the CD8α binding polypeptide may comprise the following amino acid substitutions: E30S, S60Y, E62D, S72R, Q78H, I83M, T85S, D109A, and M112L.

In some embodiments, the variable domain may be subject to humanized modification.

In some embodiments, compared with the reference antibody having the amino acid sequence as set forth in SEQ ID NO.: 1, the variable domain of the CD8α binding polypeptide may comprise amino acid substitution(s) at one or more positions selected from the group consisting of:
(a) L2; (b) A14; (c) G56; (d) N57; (e) A75; (f) K87; (g) P88; and (h) K117.

For example, compared with the reference antibody having the amino acid sequence as set forth in SEQ ID NO.: 1, the variable domain of the CD8α binding polypeptide may comprise amino acid substitutions at the following positions: (a) L2; (b) A14; (e) A75; (f) K87 and (g) P88.

For example, compared with the reference antibody having the amino acid sequence as set forth in SEQ ID NO.: 1, the variable domain of the CD8α binding polypeptide may comprise amino acid substitutions at the following positions: (a) L2; (b) A14; (e) A75; (f) K87; (g) P88; and (h) K117.

For example, compared with the reference antibody having the amino acid sequence as set forth in SEQ ID NO.: 1, the variable domain of the CD8α binding polypeptide may comprise amino acid substitutions at the following positions: (a) L2; (b) A14; (c) G56; (e) A75; (f) K87; (g) P88; and (h) K117.

For example, compared with the reference antibody having the amino acid sequence as set forth in SEQ ID NO.: 1, the variable domain of the CD8α binding polypeptide may comprise amino acid substitutions at the following positions: (a) L2; (b) A14; (d) N57; (e) A75; (f) K87; (g) P88; and (h) K117.

In some embodiments, compared with the reference antibody having the amino acid sequence as set forth in SEQ ID NO.: 1, the variable domain of the CD8α binding polypeptide may comprise one or more amino acid substitutions selected from the group consisting of:
(a) L2V; (b) A14P; (c) G56R; (d) N57R; (e) A75S; (f) K87R; (g) P88A; and (h) K117Q.

For example, compared with the reference antibody having the amino acid sequence as set forth in SEQ ID NO.: 1, the variable domain of the CD8α binding polypeptide may comprise the following amino acid substitutions: L2Y and A14P.

For example, compared with the reference antibody having the amino acid sequence as set forth in SEQ ID NO.: 1, the variable domain of the CD8α binding polypeptide further may comprise the following amino acid substitutions: A75S, K87R, P88A and/or K117Q.

For example, compared with the reference antibody having the amino acid sequence as set forth in SEQ ID NO.: 1, the variable domain of the CD8α binding polypeptide further may comprise the following amino acid substitutions: G56R and/or N57R.

For another example, compared with the reference antibody having the amino acid sequence as set forth in SEQ ID NO.: 1, the variable domain of the CD8α binding polypeptide may comprise the following amino acid substitutions: L2Y, A14P, A75S, K87R and P88A.

For another example, compared with the reference antibody having the amino acid sequence as set forth in SEQ ID NO.: 1, the variable domain of the CD8α binding polypeptide may comprise the following amino acid substitutions: L2Y, A14P, E30S, S60Y, E62D, S72R, A75S, Q78H, I83M, T85S, K87R, P88A, D109A and M112L.

In some embodiments, the variable domain may comprise an amino acid sequence as set forth in SEQ ID NO.: 14.

For example, the variable domain may comprise an amino acid sequence as set forth in SEQ ID NO.: 2, SEQ ID NO: 15 or SEQ ID NO: 16.

In some embodiments, the variable domain may comprise an amino acid sequence sharing at least about 90%, or about 91%, or about 92%, or about 93%, or about 94%, or about 95%, or about 96%, or about 97%, or about 98%, or about 99% sequence identity with SEQ ID NO.: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

For another example, the variable region may comprise an amino acid sequence as set forth in SEQ ID NO.: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

In some embodiments, the CD8α binding polypeptide may comprise one variable domain. For example, the CD8α binding polypeptide may comprise an amino acid sequence sharing at least about 90%, or about 91%, or about 92%, or about 93%, or about 94%, or about 95%, or about 96%, or about 97%, or about 98%, or about 99% sequence identity with SEQ ID NO.: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

For example, the CD8α binding polypeptide may comprise an amino acid sequence as set forth in SEQ ID NO.: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

In some embodiments, a C-terminus of the CD8α binding polypeptide may further comprise a cysteine modification.

In some embodiments, the cysteine modification may comprise the following forms: C, VDC, (GG)ₙC or (GS)ₘC, with n or m each independently selected from the group consisting of 1, 2, 3 and 4.

In some embodiments, the CD8α binding polypeptide may comprise one variable domain.

In some embodiments, the CD8α binding polypeptide may comprise an amino acid sequence as set forth in SEQ ID NO.: 29, SEQ ID NO: 31 or SEQ ID NO: 32.

### Nucleic Acid, Vector and Cell

In another aspect, the present application provides an isolated nucleic acid molecule or isolated nucleic acid molecules, encoding the CD8α binding polypeptide previously defined.

In some embodiments, the nucleic acid may encode an amino acid sequence comprising an antibody VL and/or an amino acid sequence comprising a VH (for example, a light and/or heavy chain of an antibody). In some embodiments, the nucleic acid may encode an amino acid sequence comprising an antibody VHH. In some embodiments, there is provided an isolated nucleic acid encoding a heavy-chain variable region of the anti-CD8α, where the nucleic acid comprises a sequence sharing at least about 90 %, 91 % , 92% , 93 % , 94 %, 95 %, 96 %, 97 %, 98 %, 99 % or 100% sequence identity with a nucleic acid sequence as set forth in SEQ ID NO.: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12 or SEQ ID NO: 13.

In another aspect, the present application provides a vector, comprising the nucleic acid molecule or nucleic acid molecules previously defined.

In another aspect, the present application provides a cell, comprising the nucleic acid molecule or nucleic acid molecules previously defined or the vector prevously defined.

The nucleic acid encoding the CD8α binding polypeptide of the present application may be incorporated (linked) into an expression vector, which can be then introduced into a host cell by means of transfection, transformation, or transduction techniques. For instance, the nucleic acid encoding the CD8α binding polypeptide of the present application may be introduced into the host cell by retroviral transduction. In one embodiment, the host cell is eukaryotic, for example, a Chinese hamster ovary (CHO) cell or a lymphoid cell (for example, a Y0, NS0, or Sp20 cell). In one embodiment, the host cell is prokaryotic, for example, an E. coli cell.

In another aspect, the present application provides a method for producing a CD8α binding polypeptide. The method may comprise culturing the above-mentioned cell comprising an antibody-encoding nucleic acid under a condition suitable for expression of the CD8α binding polypeptide, and optionally, recovering the CD8α binding polypeptide from the cell (or a cell culture liquid).

In some embodiments, the method may further comprise purifying and/or modifying the CD8α binding polypeptide.

The specific expression and purification conditions vary depending on the expression system used. For instance, if a gene is to be expressed in E. coli, the engineered gene is first placed downstream of a suitable bacterial promoter (for example, Trp or Tac) and a prokaryotic signal sequence, and is then cloned to an expression vector. In another instance, if an engineered gene is to be expressed in a eukaryotic cell (for example, a CHO cell), the gene is first inserted into an expression vector comprising, for example, an eukaryotic promoter, a secretion signal, an enhancer, and various introns as appropriate. A gene construct may be introduced into a host cell by using transfection, transformation, or transduction techniques.

The CD8α binding polypeptide of the present application may also be expressed in vivo, for example, in a patient. For instance, in some embodiments, the CD8α binding polypeptide of the present application may be administered in the form of a nucleic acid encoding the CD8α binding polypeptide of the present application. The nucleic acid may be DNA or RNA. In some embodiments, the CD8α binding polypeptide of the present application is encoded by a modified mRNA, i.e., an mRNA comprising one or more modified nucleotides. In some embodiments, the present application relates to a gene therapy vector comprising the modified mRNA. In some embodiments, the present application relates to a gene therapy method comprising the gene therapy vector. In some embodiments, the nucleic acid is in the form of an oncolytic virus, for example, an adenovirus, a reovirus, measles, herpes simplex, a Newcastle disease virus, or vaccinia.

### Immunoconjugate

In another aspect, the present application provides an immunoconjugate, comprising the CD8α binding polypeptide previously defined.

In some embodiments, the immunoconjugate further comprises at least one detectable marker binding to the CD8α binding polypeptide.

In some embodiments, the detectable marker may be selected from the group consisting of a radionuclide, a fluorescent agent, a chemiluminescent agent, a bioluminescent agent, a paramagnetic ion, and an enzyme.

A fluorescent agent available for conjugation includes, but is not limited to, fluorescein isothionine, rhodamine, phycoerythrin, phycocyanin, allophycocyanin, o-phthalaldehyde, and fluorescamine; a chemiluminescent agent available for conjugation includes, but is not limited to, luminol, isoluminol, aromatic acridinium ester, imidazoles, acridinium salts, and oxalates; and a bioluminescent agents available for conjugation includes, but is not limited to, luciferin, luciferase, and aequorin. Paramagnetic ions available for conjugation include, but are not limited to, chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III), and erbium(III), or radiopaque materials such as barium, diatrizoate, ethiodized-oil, gallium citrate, iocarmic acid, iocetamic acid, iodamide, iodipamide, iodoxamic acid, iodoglutamide, iohexol, iopamidol, iopanoic acid, ioprocemic acid, iosefamic acid, ioseric acid, meglumine iodosulfone, iotasul, iotetric acid, iotalamic acid, iotroxic acid, ioxaglic acid, ioxotrizoic acid, ipodate, meglumine, metrizoate , metrizamide, propidium iodide and thallous oxide. Enzymes available for conjugation include, but are not limited to, horse radish peroxidase or the like.

In some embodiments, the detectable marker may be a radionuclide. The radionuclide available for conjugation may be a radionuclide having the energy ranging between 20 KeV and 4000KeV, including but not limited to ¹¹⁰In, ¹¹¹In, ¹⁷⁷Lu, ¹⁸F, ⁵²Fe, ⁶²Cu, ⁶⁷Cu, ⁶⁷Ga, ⁶⁸Ga, ⁶⁸Ge, ⁸⁶Y, ⁹⁰Y, ⁸⁹Zr, ^{94m}Tc, ¹²⁰I, ¹²³I, ¹²⁴I, ¹²⁵I, ¹³¹I, ¹⁵⁴⁻¹⁵⁸Gd, ³²P, ¹¹C, ¹³N, ¹⁵O, ¹⁸⁶Re, ¹⁸⁸Re, ⁵¹Mn, ^{52m}Mn, ⁷²As, ⁷⁵Br, ⁷⁶Br, ^{82m}Rb, ⁸³Sr or other γ-, β-, or positron-emitters.

In some embodiments, the detectable marker may be ⁶⁸Ga or ¹²⁵I.

The method of conjugating a detectable label to a polypeptide is well known to those skilled in the art. For example, in some embodiments, the CD8α binding polypeptide may be conjugated with the detectable marker via a chelating agent.

In some embodiments, in order to label the CD8α binding polypeptide of the present application with a radionuclide such as 68Ga, it is necessary to react the CD8α binding polypeptide of the present application with a reagent having a long tail, to which a plurality of integrated groups is attached for binding to ions. Such a tail may be, for example, polylysine, polysaccharide, or other polymers with derivatized or derivatable chains having side groups that is capable of binding to a chelating group, for example, ethylenediamine tetraacetic acid (EDTA), diethylene triamine pentaacetic acid (DTPA), DOTA (1,4,7,10-tetraazacyclododecane-1,4,7,10-tetracarboxylic acid), NOTA, TETA, NETA, porphyrins, polyamines, coronal ethers, diaminothiourea, polyoxime, and similar groups known to be used for this purpose.

In some embodiments, the detectable marker may be conjugated with the CD8α binding polypeptide of the present application via a chelating agent. The chelating agent used comprises, but is not limited to, DTPA, EDTA, NOTA, DOTA, TRAP, TETA, NETA, CB-TE2A, Cyclen, Cyclam, Bispidine, TACN, ATSM, SarAr, AmBaSar, MAG3, MAG2, HYNIC, DADT, EC, NS3, H2dedpa, HBED, DFO, PEPA or HEHA, or their derivatives.

In some embodiments, the detectable marker may be ⁶⁸Ga.

In some embodiments, the chelating agent may be NOTA or a derivative thereof. For example, the detectable marker may be ⁶⁸Ga, and the chelating agent may be NOTA. For example, the detectable marker may be 68Ga, and the chelating agent may be NODAGA.

In another aspect, the present application provides a method for preparing a radionuclide (such as 68Ga)-labeled immunoconjugate of the present application, comprising 1) conjugating the CD8α binding polypeptide of the present application with a chelating agent to produce a conjugate of the CD8α binding polypeptide and the chelating agent; and 2) contacting the product of step 1) with a radionuclide such as 68Ga, whereby the CD8α binding polypeptide of the present application is labeled with the radionuclide such as 68Ga by means of the chelating agent. In some embodiments, the chelating agent is NOTA, and in step 1), a conjugate of the CD8-binding polypeptide and NOTA is produced by reacting the CD8α binding polypeptide with p-SCN-Bn-NOTA or p-NH2-Bn-NOTA. In other embodiments, the chelating agent is NODAGA, and in step 1), a conjugate of the CD8-binding polypeptide and NODAGA is produced by reacting the CD8α binding polypeptide with Maleimide-NODAGA.

In another aspect, the present application provides a method for preparing a 125I-labeled conjugated molecule of the present application, comprising: 1) reacting the CD8α binding polypeptide of the present application with 125I in the presence of chloramine T; and 2) stopping the reaction with a sodium metabisulfite.

### Composition

In another aspect, the present application provides a composition, comprising the CD8α binding polypeptide previously defined, the nucleic acid molecule or nucleic acid molecules previously defined, the vector previously defined, the cell previously defined and/or the immunoconjugate previously defined, and optionally, a pharmaceutically acceptable carrier.

The CD8α binding polypeptide described in the present application may have a sufficiently basic functional group capable of reacting with an inorganic or organic acid, or a carboxyl group capable of reacting with an inorganic or organic base, to form a pharmaceutically acceptable salt. As is well known in the art, a pharmaceutically acceptable acid addition salt is formed from a pharmaceutically acceptable acid. Such a salt includes, for example, the pharmaceutically acceptable salts listed in the following literature: Journal of Pharmaceutical Science, 66, 2-19 (1977); and The Handbook of Pharmaceutical Salts; Properties, Selection, and Use. P. H. Stahl and C.G. Wermuth (eds.), Verlag, Zurich (Switzerland) 2002, which are all incorporated into the present application in their entireties by reference. In some embodiments, the composition described in the present application is in the form of a pharmaceutically acceptable salt.

Any composition described in the present application may be administered to a subject as a component of the composition comprising the pharmaceutically acceptable carrier. Such a composition may optionally comprise an appropriate amount of a pharmaceutically acceptable excipient, in order to provide a form for proper administration.

The pharmaceutical excipient may be a liquid, such as water and oil, including those of petroleum, animal, vegetable, or synthetic sources, such as peanut oil, soybean oil, mineral oil, sesame oil or the like. The pharmaceutical excipient may be, for example, normal saline, gum arabic, gelatin, starch paste, talc, keratin, colloidal silica, urea or the like. In addition, an additive, a stabilizer, a thickener, a lubricant and a colorant may be used. In one embodiment, the pharmaceutically acceptable excipient is sterile when administered to a subject. Water is an available excipient when any of the agents described in the present application is administered intravenously. A normal saline solution and dextrose water and glycerite may also be used as liquid excipients, specifically, for injectable solutions. Suitable pharmaceutical excipients further include starch, glucose, lactose, sucrose, gelatin, malt, rice, flour, chalk, silica gel, sodium stearate, glyceryl monostearate, talc, sodium chloride, skimmed milk powder, glycerin, propylene, ethylene glycol, water, ethanol and the like. Other examples of the suitable pharmaceutical excipients are described in Remington's Pharmaceutical Sciences 1447-1676 (Alfonso R. Gennaro ed., 19th Edition, 1995), which is incorporated into the present application by reference.

The present application comprises various forms of formulations of the described composition (and/or other therapeutic agents). Any inventive composition (and/or other therapeutic agents) described in the present application may be in the form of a solution, a suspension, an emulsion, drops, tablets, pills, sublimed preparations, capsules, capsules comprising liquids, gelatin capsules, powder, sustained-release formulations, suppositories, emulsions, aerosols, sprays, suspensions, lyophilized powder, frozen suspensions, dry powder or their any other combinations. In one embodiment, the composition is in the form of a capsule. In another embodiment, the composition is in the form of a lozenge. In another embodiment, the composition is formulated in the form of a soft gel capsule. In another embodiment, the composition is formulated in the form of a gelatin capsule. In another embodiment, the composition is formulated as a liquid.

Any composition described in the present application is formulated according to conventional procedures into a composition suitable for the mode of administration described in the present application.

Routes of administration include, for example: oral, intradermal, intramuscular, intraperitoneal, intravenous, subcutaneous, intranasal, epidural, sublingual, intranasal, intraceretal, intrathecal, percutaneous, rectal, inhalation, or topical administration. The administration may be topical or systemic. In some embodiments, the administrating is implemented orally. In another embodiment, the administration is parenteral injection. The mode of administration may be left to the physician's judgment and depends in part on the location of a medical condition.

In some embodiments, the composition may comprise the immunoconjugate of the present application, and the composition may be a detecting agent. In some embodiments, the detecting agent may be a contrast agent. For example, the contrast agent may be an ECT contrast agent. For another example, the ECT contrast agent may comprise an SPECT contrast agent or a PET contrast agent.

### Uses in Detection/Diagnosis

In another aspect, the present application provides use of the CD8α binding polypeptide previously defined, the nucleic acid molecule or nucleic acid molecules previously defined, the vector previously defined, the cell previously defined, the immunoconjugate previously defined, and/or the composition previously defined in preparation of a medicament for monitoring, preventing, alleviating and/or treating tumors.

In another aspect, the present application provides use of the CD8α binding polypeptide of the present application or the immunoconjugate of the present application in preparation of a detecting agent for detecting CD8-positive cells. In some embodiments, the detecting agent may be used to detect the presence and/or amount of CD8-positive cells in tissues in an object in vivo. In some embodiments, the tissues are tumor tissues. In some embodiments, the CD8-positive cells may be CD8-positive T cells. In some embodiments, the detecting agent may be a contrast agent. In some embodiments, the contrast agent may be an ECT contrast agent, for example, an SPECT contrast agent or a PET contrast agent.

In another aspect, the present application provides a method for detecting presence and/or amount of CD8 in a biological sample, comprising contacting the biological sample with the CD8α binding polypeptide previously defined, the immunoconjugate previously defined, the composition previously defined or the detecting agent previously defined.

In some embodiments, the contacting may be conducted in vitro or ex vivo.

In some embodiments, the biological sample may be a tissue.

In some embodiments, the tissue may be selected from the group consisting of a blood tissue, a lymphoid tissue and a tumor tissue.

In some embodiments, the method may comprise detecting presence and/or amount of CD8-positive cells in the biological sample.

In some embodiments, the presence and/or amount of the CD8-positive cells in the biological sample may be determined by imaging.

In some embodiments, the presence and/or amount of the CD8-positive cells in the biological sample may be determined by flow cytometry.

In some embodiments, the CD8-positive cells may be CD8-positive T cells.

In another aspect, the present application provides a method for detecting and/or diagnosing CD8-related diseases or disorders. The method may comprise administering the CD8α binding polypeptide previously defined, the immunoconjugate previously defined, the composition previously defined or the detecting agent previously defined to a subject in need thereof.

In some embodiments, the method may further comprise performing imaging on the subject. In some embodiments, the imaging may comprise ECT imaging.

In some embodiments, the ECT imaging may comprise SPECT imaging or PET imaging. In some embodiments, the CD8-related diseases or disorders may comprise tumors.

In another aspect, the present application provides a method for treating tumors. The method may comprise:
1) administering the CD8α binding polypeptide previously defined, the immunoconjugate previously defined, the composition previously defined or the detecting agent previously to a subject in need thereof; and
2) determining whether a tumor in the subject comprises CD8-positive cells,
wherein if presence of the CD8-positive cells in the tumor is detected, an anti-tumor therapy is administered to the subject.

In another aspect, the present application provides a method for monitoring efficacy of an anti-tumor therapy in a subject. The method may comprise:
1) administering the CD8α binding peptide previously defined, the immunoconjugate previously defined, the composition previously defined or the detecting agent previously to the subject suffering from a tumor and treated with the anti-tumor therapy; and
2) determining an amount of CD8-positive cells in a tumor of the subject.

In some embodiments, the presence and/or amount of the CD8-positive cells in the tumor of the subject is determined by imaging.

In some embodiments, the anti-tumor therapy is an immune checkpoint inhibitor therapy.

In some embodiments, the anti-tumor therapy may be selected from administration of a PD-1 inhibitor, a PD-L1 inhibitor, a CTLA-4 inhibitor, a TIM3 inhibitor, a BTLA inhibitor, a TIGIT inhibitor, a CD47 inhibitor, a GITR inhibitor, an LAG3 inhibitor, an additional T cell co-inhibitor or a ligand antagonist, an indolamine-2,3-dioxygenase IDO inhibitor, a vascular endothelial growth factor (VEGF) antagonist, an Ang2 inhibitor, a transforming growth factor β (TGFβ) inhibitor, an epidermal growth factor receptor (EGFR) inhibitor, a CD20 inhibitor, an antibody or a vaccine for a tumor-specific antigen, an adjuvant to increase antigen presentation, a bispecific antibody, a cytotoxin, a chemoagent, cyclophosphamide, a radiotherapy, an IL-6R inhibitor, an IL-4R inhibitor, an IL-10 inhibitor, a cytokine, and an antibody-drug conjugate (ADC).

In some embodiments, the tumor may comprise a solid tumor.

In some embodiments, the solid tumor may be selected from the group consisting of a colorectal cancer, an ovarian cancer, a prostate cancer, a breast cancer, a brain cancer, a cervical cancer, a bladder cancer, an anal cancer, an uterine cancer, a colon cancer, a liver cancer, a pancreatic cancer, a lung cancer, an endometrial cancer, a bone cancer, a testicular cancer, a skin cancer, a renal cancer, a gastric cancer, an esophageal cancer, a head and neck cancer, a salivary gland cancer and myeloma.

In another aspect, the present application provides a method for isolating CD8-positive cells. The method may comprise: contacting a cell population comprising CD8-positive cells with the CD8α binding polypeptide previously defined or the immunoconjugate previously defined, and recovering CD8-positive cells binding to the CD8α binding polypeptide previously defined or the immunoconjugate previously defined.

In some embodiments, the CD8-positive cells may be CD8-positive T cells.

In some embodiments, the cell population comprising CD8-positive cells may be human peripheral blood mononuclear cells (PBMCs).

In some embodiments, the CD8α binding polypeptide previously defined or the immunoconjugate previously defined may be immobilized on a surface of a solid.

In some embodiments, the solid may comprise a gel or a magnetic bead.

In another aspect, the present application provides a kit, which may comprise the CD8α binding polypeptide previously defined, the immunoconjugate previously defined or the composition previously defined.

Instructions for use may be included in the kit. The instructions for use typically include tangible representations that describe the desired therapeutic outcome achieved by using the components of the kit, such as the technology to be used in the treatment of a cancer. Optionally, the kit also comprises other useful components that are readily known to those skilled in the art, such as diluents, buffers, pharmaceutically acceptable carriers, syringes, catheters, applicators, pipet or measurement tools, bandaging materials, or other useful accessories.

The materials and components assembled in the kit may be provided to practitioners, and stored in any convenient and appropriate manner that maintains their operability and utility. For example, these components may be provided at room temperature, refrigerated temperature, or frozen temperature. These components are typically included in a suitable packaging material. In some embodiments, the packaging material is constructed by well-known methods, preferably to provide a sterile and contamination-free environment. The packaging material may have an external label indicating the contents and/or purposes of the kit and/or its components.

Not wishing to be bound by any particular theory, the following examples are merely to illustrate the antibody, its preparation method and uses or the like of the present application, and are not intended to limit the scope of the present invention. In the present application, a CD8 single-domain antibody or a CD8α single-domain antibody is disclosed as in CN202010703962.9, which is incorporated into the present application by reference in its entirety.

### Examples

### Example 1

### 1.1 Sequence optimization for affinity packing-binding fraction of C37 antibody

After preliminary screening and identification, C37-cHis was selected as a final antibody sequence (including a histidine tag) from candidate antibodies, and meanwhile, an untagged recombinant plasmid C37 was constructed. HEK293 cells were transfected with the single-domain antibody C37 fusion protein plasmid constructed by the above recombination for expression of an antibody. Moreover, PROTEIN A affinity chromatography was conducted for purification, and it was found by identification that the C37 antibody did not bind to the packing of the PROTEIN A (ProteinA) affinity chromatography, which increased the difficulty in obtaining a protein by subsequent purification. Therefore, it was necessary to optimize the sequence of the C37 antibody to allow purification of the C37 antibody by affinity chromatography.

The FR (Framework region) of the sequence of the C37 antibody was analyzed; key amino acids associated with the binding to the PROTEIN A affinity packing were selected for mutation; and after several rounds of mutation, a final mutant C37-YDHMS could bind to the packing of the PROTEIN A affinity chromatography.

Nomenclature of mutants and amino acid sequences

| Designatio n | Sequence | Bind ing or not |
|---|---|---|
| C37-S60Y | QLQLVESGGGLVQAGGSLRLSCAASGLTFEDYAIGW FRQAPGKEREGISCIRIYDGNTYYAESVKGRFTISSDNAKN QVYLQINTLKPEDTAVYYCAAGSYYSCSVYPDYDMDYW GKGTLVTVSS | Not binding |
| C37-T85S | QLQLVESGGGLVQAGGSLRLSCAASGLTFEDYAIGW FRQAPGKEREGISCIRIYDGNTYSAESVKGRFTISSDNAKN QVYLQINSLKPEDTAVYYCAAGSYYSCSVYPDYDMDYW GKGTLVTVSS | Not binding |
| C37-ST-Y S | QLQLVESGGGLVQAGGSLRLSCAASGLTFEDYAIGW FRQAPGKEREGISCIRIYDGNTYYAESVKGRFTISSDNAKN QVYLQINSLKPEDTAVYYCAAGSYYSCSVYPDYDMDYW GKGTLVTVSS | Not binding |
| C37-YDS | QLQLVESGGGLVQAGGSLRLSCAASGLTFEDYAIGW FRQAPGKEREGISCIRIYDGNTYYADSVKGRFTISSDNAK NQVYLQINSLKPEDTAVYYCAAGSYYSCSVYPDYDMDY WGKGTLVTVSS | Not binding |
| C37-YMS | QLQLVESGGGLVQAGGSLRLSCAASGLTFEDYAIGW FRQAPGKEREGISCIRIYDGNTYYAESVKGRFTISSDNAKN | Not binding |
| | QVYLQMNSLKPEDTAVYYCAAGSYYSCSVYPDYDMDY WGKGTLVTVSS | |
| C37-YDM S | QLQLVESGGGLVQAGGSLRLSCAASGLTFEDYAIGW FRQAPGKEREGISCIRIYDGNTYYADSVKGRFTISSDNAK NQVYLQMNSLKPEDTAVYYCAAGSYYSCSVYPDYDMD YWGKGTLVTVSS | Not binding |
| C37-YDH MS | QLQLVESGGGLVQAGGSLRLSCAASGLTFEDYAIGW FRQAPGKEREGISCIRIYDGNTYYADSVKGRFTISSDNAK NHVYLQMNSLKPEDTAVYYCAAGSYYSCSVYPDYDMD YWGKGTLVTVSS | Bind ing |

| | | |
|---|---|---|
| Note: The underlined part indicates a mutational site. | | |

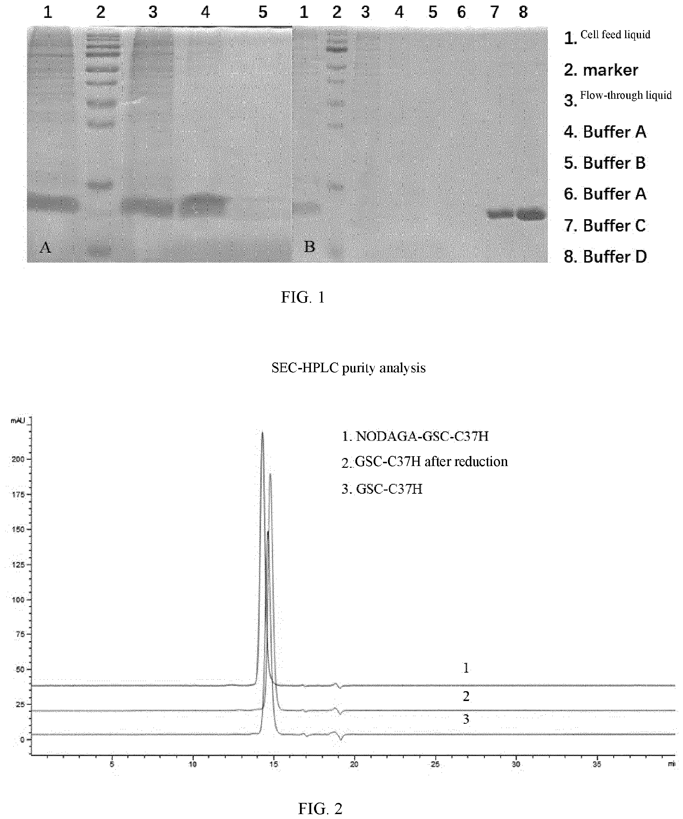

The recombinant plasmid C37 and the mutated recombinant plasmid C37-YDHMS were subjected to antibody expression via the HEK293 cells, respectively, and purification was conducted by means of the packing of the PROTEIN A affinity chromatography, in order to verify the binding to the affinity packing after the mutations. FIG. 1 shows SDS-PAGE analysis results after antibody purification before and after the mutations. FIG. 1A shows the SDS-page plot of C37 purification. As shown in FIG. 1A, C37 does not bind to the PROTEIN A packing, and some of the proteins of interest directly flow through in the flow-through liquid, and some of the proteins of interest are washed out in the equilibrium buffer A. FIG. 1B shows C37-YDHMS. As shown in FIG> 1B, the proteins of interest are not found in both the flow-through liquid and the equilibrium buffers A and B, and pure proteins of interest are found in the elution buffers C and D, indicating normal binding of the C37-YDHMS to the PROTEIN A packing after modification.

### 1.2 Sequence optimization of C37 antibody in terms of binding activity, expressing level and PI value

For an antibody molecule, an isoelectric point (PI) was generally required to be higher than 7, in order to ensure that the molecule was suitable for ion-exchange chromatography in a downstream purification process platform. Moreover, the isoelectric point of a host DNA was mainly distributed in a range of 4-4.5, and it was difficult to achieve a removal effect if the PI value of the antibody is close to it. The PI value of the C37 antibody was 4.56, which is close to the isoelectric point of DNA, anticipating a poor removal effect. Meanwhile, to make it easier to select the ion-exchange packing for downstream purification, it is necessary to perform mutating modification on the sequence of the C37 antibody, in order to increase the PI value.

The FR and CDR (complementarity-determining region) of the sequence of the C37 antibody were analyzed, and the amino acids associated with affinity, expression level and PI value were selected for mutation attempt. A single amino acid was first selected for mutation, then multiple amino acids were subjected to mutations, and a total of 15 groups of mutations were performed on the fractions associated with the expression level and binding activity.

Nomenclature of mutants and amino acid sequences

| Designatio n | Sequence |
|---|---|
| C37-S72R | QLQLVESGGGLVQAGGSLRLSCAASGLTFEDYAIGWFRQAPG KEREGISCIRIYDGNTYSAESVKGRFTISRDNAKNQVYLQINTLKPE DTAVYYCAAGSYYSCSVYPDYDMDYWGKGTLVTVSS |
| C37-KL-Q Q | QLQLVESGGGLVQAGGSLRLSCAASGLTFEDYAIGWFRQAPG KEREGISCIRIYDGNTYSAESVKGRFTISSDNAKNQVYLQINTLKPED TAVYYCAAGSYYSCSVYPDYDMDYWGQGTQVTVSS |
| C37-D109 A | QLQLVESGGGLVQAGGSLRLSCAASGLTFEDYAIGWFRQAPG KEREGISCIRIYDGNTYSAESVKGRFTISSDNAKNQVYLQINTLKPED TAVYYCAAGSYYSCSVYPAYDMDYWGKGTLVTVSS |
| C37-D111 A | QLQLVESGGGLVQAGGSLRLSCAASGLTFEDYAIGWFRQAPG KEREGISCIRIYDGNTYSAESVKGRFTISSDNAKNQVYLQINTLKPED TAVYYCAAGSYYSCSVYPDYAMDYWGKGTLVTVSS |
| C37-D31Y | QLQLVESGGGLVQAGGSLRLSCAASGLTFEYYAIGWFRQAPG KEREGISCIRIYDGNTYSAESVKGRFTISSDNAKNQVYLQINTLKPED TAVYYCAAGSYYSCSVYPDYDMDYWGKGTLVTVSS |
| C37-T85S | QLQLVESGGGLVQAGGSLRLSCAASGLTFEDYAIGWFRQAPG KEREGISCIRIYDGNTYSAESVKGRFTISSDNAKNQVYLQINSLKPED TAVYYCAAGSYYSCSVYPDYDMDYWGKGTLVTVSS |
| C37-M112 L | QLQLVESGGGLVQAGGSLRLSCAASGLTFEDYAIGWFRQAPG KEREGISCIRIYDGNTYSAESVKGRFTISSDNAKNQVYLQINTLKPED TAVYYCAAGSYYSCSVYPDYDLDYWGKGTLVTVSS |
| C37-TMS | QLQLVESGGGLVQAGGSLRLSCAASGLTFEDYAIGWFRQAPG KEREGISCIRIYDGNTYSAESVKGRFTISSDNAKNTVYLQMNSLKPE |
| | DTAVYYCAAGSYYSCSVYPDYDMDYWGKGTLVTVSS |
| C37-E30S | QLQLVESGGGLVQAGGSLRLSCAASGLTFSDYAIGWFRQAPG KEREGISCIRIYDGNTYSAESVKGRFTISSDNAKNQVYLQINTLKPED TAVYYCAAGSYYSCSVYPDYDMDYWGKGTLVTVSS |
| C37-RA | QLQLVESGGGLVQAGGSLRLSCAASGLTFEDYAIGWFRQAPG KEREGISCIRIYDGNTYSAESVKGRFTISRDNAKNQVYLQINTLKPE DTAVYYCAAGSYYSCSVYPAYDMDYWGKGTLVTVSS |
| C37-SA | QLQLVESGGGLVQAGGSLRLSCAASGLTFEDYAIGWFRQAPG KEREGISCIRIYDGNTYSAESVKGRFTISSDNAKNQVYLQINSLKPED TAVYYCAAGSYYSCSVYPAYDMDYWGKGTLVTVSS |
| C37-SR | QLQLVESGGGLVQAGGSLRLSCAASGLTFSDYAIGWFRQAPG KEREGISCIRIYDGNTYSAESVKGRFTISRDNAKNQVYLQINTLKPE DTAVYYCAAGSYYSCSVYPDYDMDYWGKGTLVTVSS |
| C37-RSA | QLQLVESGGGLVQAGGSLRLSCAASGLTFEDYAIGWFRQAPG KEREGISCIRIYDGNTYSAESVKGRFTISRDNAKNQVYLQINSLKPED TAVYYCAAGSYYSCSVYPAYDMDYWGKGTLVTVSS |
| C37-SRA | QLQLVESGGGLVQAGGSLRLSCAASGLTFSDYAIGWFRQAPG KEREGISCIRIYDGNTYSAESVKGRFTISRDNAKNQVYLQINTLKPE DTAVYYCAAGSYYSCSVYPAYDMDYWGKGTLVTVSS |
| C37-SRS | QLQLVESGGGLVQAGGSLRLSCAASGLTFSDYAIGWFRQAPG KEREGISCIRIYDGNTYSAESVKGRFTISRDNAKNQVYLQINSLKPED TAVYYCAAGSYYSCSVYPDYDMDYWGKGTLVTVSS |

| | |
|---|---|
| Note: The underlined part indicates a mutated amino acid. | |

The expression level and binding activity of each mutant were separately detected. For the sake of detection, a histidine-tagged recombinant plasmid was used in this section for transient transfection, after which a recombinant protein was purified by affinity chromatography with a corresponding Ni+ resin gel, thereby obtaining a protein of interest for direct use in the detection of expression level and binding activity.

Detection of affinity activity: a CD8α Fc fusion protein was used to coat a plate at 0.5 µg/well at 4°C overnight; PBST washing conducted and then 3% BSA was added for blocking; and then a series of serially diluted C37 single-domain antibody proteins of each mutant were added to allow reaction for 1 hour at room temperature. After washing, an anti-His horseradish peroxidase-labeled antibody was added to allow reaction for 1 hour at room temperature. After washing, a chromogenic solution was added, and an absorption value was read at a wavelength of 450 nm. The software SotfMaxPro v5.4 was used for data processing and diagraph analysis; four-parameter fitting was performed to obtain the binding curve of the mutated antibody to CD8α; and different mutants were compared in relative activity with the C37 antibody as a control, in order to reflect the affinity of the antibody to CD8α.

Calculation of expression level: The expression level was calculated based on the total amount of proteins of interest obtained after one-step purification by affinity chromatography.

**Table 1. Binding of each mutant to CD8α and expression level**

| Designation | Relative activity (%) | Expression level (g/L) |
|---|---|---|
| C37 | 100 | 0.073 |
| C37-S72R | 115 | 0.106 |
| C37-KL-QQ | 141 | 0.101 |
| C37-D109A | 476 | 0.054 |
| C37-D111A | 84.6 | 0.037 |
| C37-D31Y | 48.4 | 0.017 |
| C37-T85S | 162 | 0.107 |
| C37-M112L | 206 | 0.011 |
| C37-TMS | 107 | 0.004 |
| C37-E30S | 126 | 0.020 |
| C37-RA | 108 | 0.092 |
| C37-SA | 257 | 0.063 |
| C37-SR | 42 | 0.036 |
| C37-RSA | 103 | 0.098 |
| C37-SRA | 40 | 0.033 |
| C37-SRS | 33 | 0.043 |

The affinity of each mutant with a CD8α antigen and the expression level were combined for analysis. In this section, the mutants selected for combination included C37-S72R, C37-T85S and C37-D109A, that is, C37-RSA was the final mutant in this section.

Mutation points associated with the purification of affinity chromatography and mutation points associated with the affinity and expression level were combined; on this basis, negatively-charged amino acids in the antibody sequence were continuously analyzed; and generalized analysis was performed according to the overall situation of the sequence, and the negatively-charged amino acids were mutated, in order to increase the overall PI value of the antibody.

Nomenclature of mutants and amino acid sequences

| Designation | Sequence | Theoret ical PI value |
|---|---|---|
| C37-R | QLQLVESGGGLVQAGGSLRLSCAASGLTFEDYA IGWFRQAPGKEREGISCIRIYDGNTYYADSVKGRFTIS RDNAKNQVYLQMNSLKPEDTAVYYCAAGSYYSCSV YPDYDMDYWGKGTLVTVSS | 4.98 |
| C37-TRA | QLQLVESGGGLVQAGGSLRLSCAASGLTFEDYA IGWFRQAPGKEREGISCIRIYDGNTYYADSVKGRFTIS RDNAKNTVYLQMNSLKPEDTAVYYCAAGSYYSCSV YPAYDMDYWGKGTLVTVSS | 5.30 |
| C37-YDMS-A | QLQLVESGGGLVQAGGSLRLSCAASGLTFEDYA IGWFRQAPGKEREGISCIRIYDGNTYYADSVKGRFTIS SDNAKNQVYLQMNSLKPEDTAVYYCAAGSYYSCSV YPAYDMDYWGKGTLVTVSS | 4.96 |
| C37-YDTMS -A | QLQLVESGGGLVQAGGSLRLSCAASGLTFEDYA IGWFRQAPGKEREGISCIRIYDGNTYYADSVKGRFTIS SDNAKNTVYLQMNSLKPEDTAVYYCAAGSYYSCSV YPAYDMDYWGKGTLVTVSS | 4.96 |
| C37-YDTMS -R | QLQLVESGGGLVQAGGSLRLSCAASGLTFEDYA IGWFRQAPGKEREGISCIRIYDGNTYYADSVKGRFTIS RDNAKNTVYLQMNSLKPEDTAVYYCAAGSYYSCSV YPDYDMDYWGKGTLVTVSS | 4.98 |
| C37-YDMS-SR | QLQLVESGGGLVQAGGSLRLSCAASGLTFSDYA IGWFRQAPGKEREGISCIRIYDGNTYYADSVKGRFTIS RDNAKNQVYLQMNSLKPEDTAVYYCAAGSYYSCSV YPDYDMDYWGKGTLVTVSS | 5.27 |
| C37-YDMS-SRA | QLQLVESGGGLVQAGGSLRLSCAASGLTFSDYA IGWFRQAPGKEREGISCIRIYDGNTYYADSVKGRFTIS RDNAKNQVYLQMNSLKPEDTAVYYCAAGSYYSCSV YPAYDMDYWGKGTLVTVSS | 6.37 |
| C37-F | QLQLVESGGGLVQAGGSLRLSCAASGLTFSDYA IGWFRQAPGKEREGISCIRIYDGNTYYADSVKGRFTIS RDNAKNTVYLQMNSLKPEDTAVYYCAAGSYYSCSV YPAYDLDYWGKGTLVTVSS | 6.37 |
| C37-F1 | QLQLVESGGGLVQAGGSLRLSCAASGLTFSDYA IGWFRQAPGKEREGISCIRIYDGNTYYADSVKGRFTIS RDNAKNHVYLQMNSLKPEDTAVYYCAAGSYYSCSV YPAYDLDYWGKGTLVTVSS | 6.75 |
| C37-F2 | QLQLVESGGGLVQAGGSLRLSCAASGLTFSDYA IGWFRQAPGKEREGISCIRIYDGNTYYADSVKGRFTIS RDNAKNQVYLQMNSLKPEDTAVYYCAAGSYYSCSV YPAYDLDYWGKGTLVTVSS | 6.37 |
| C37-F4 | QVQLVESGGGLVQAGGSLRLSCAASGLTFSDYA IGWFRQAPGKEREGISCIRIYDGNTYYADSVKGRFTIS RDNAKNTVYLQMNSLKPEDTAVYYCAAGSYYSCSV YPAYDLDYWGKGTLVTVSS | 6.37 |
| C37-F5 | QVQLVESGGGLVQPGGSLRLSCAASGLTFSDYA IGWFRQAPGKEREGISCIRIYDGNTYYADSVKGRFTIS RDNAKNTVYLQMNSLKPEDTAVYYCAAGSYYSCSV YPAYDLDYWGKGTLVTVSS | 6.37 |

| | | |
|---|---|---|
| Note: The underlined part indicates a mutated amino acid. | | |

After a series of mutating modifications to the sequence of the C37 single-domain antibody, C37-F 1 was finally determined as the final mutant of this stage. Compared with the original C37 single-domain antibody before the modification, the PI value was increased from 4.56 to 6.75.

### 1.3 Humanization of C37-F1 single-domain antibody

A camel antibody had advantages that traditional antibodies did not have, such as small molecular weight, good permeability in vivo, and readiness to pass through blood vessels or tissues to reach a target site. These advantages allowed nanobodies to be widely used as tools for disease diagnosis and detection. However, if multivalent antibodies were used in clinical practice for a long time, they might produce immune responses to different extents and affect the therapeutic effect. Therefore, it was necessary to humanize the C37-F1 antibody. Humanization was completed by a method for humanizing amino acids on the surface of a protein and a method for transplanting a humanized general framework fraction of VHH.

Humanization steps were as follows: acquiring a general humanized VHH framework h-NbBcIII0FGLA (PDB NO.: 3EAK) designed by Cecile Vincke et al. according to sequence homology, with the framework designed based on a nanobody NbBcIII0 antibody (PDB NO.: 3DWT); humanizing amino acids on the surface of the protein of a reference humanized antibody; and selecting a site to be humanized according to the specific situation of the C37-F1 nanobody sequence.

The C37-F1 antibody was humanized, and a total of 5 humanized variants of the antibody strain C37-F1 were obtained. Table 2 lists the amino acid changes of these humanized variants and the nomenclature of the mutated antibodies, in which the amino acid residue number is according to the Kabat number, and the humanized variant C37H was finally selected as the mutated antibody after humanization.

**Table 2. Correlation table of amino acid mutations**

| | **L2** | **A1** | **G** | **N57R** | **A7** | **K8** | **P8** | **K1** |
|---|---|---|---|---|---|---|---|---|
| | **V** | **4P** | **56R** | | **5S** | **7R** | **8A** | **17Q** |
| **C37H** | √ | √ | | | √ | √ | √ | |
| **C37H1** | √ | √ | | √ | √ | √ | √ | √ |
| **C37H2** | √ | √ | √ | | √ | √ | √ | √ |
| **C37H3** | √ | √ | | | | √ | √ | |
| **C37H4** | √ | √ | | | √ | | | |

The specific designations and amino acid sequences of the mutants are as follows:

| Design ation | Sequence | PI value |
|---|---|---|
| C37H | QVQLVESGGGLVQPGGSLRLSCAASGLTFSDYAIG WFRQAPGKEREGISCIRIYDGNTYYADSVKGRFTISRDN | 6.75 |
| | SKNHVYLQMNSLRAEDTAVYYCAAGSYYSCSVYPAYD LDYWGKGTLVTVSS | |
| C37-H 1 | QVQLVESGGGLVQPGGSLRLSCAASGLTFSDYAIG WFRQAPGKEREGISCIRIYDGRTYYADSVKGRFTISRDN SKNHVYLQMNSLRAEDTAVYYCAAGSYYSCSVYPAYD LDYWGQGTLVTVSS | 6.75 |
| C37-H 2 | QVQLVESGGGLVQPGGSLRLSCAASGLTFSDYAIG WFRQAPGKEREGISCIRIYDRNTYYADSVKGRFTISRDN SKNHVYLQMNSLRAEDTAVYYCAAGSYYSCSVYPAYD LDYWGQGTLVTVSS | 6.75 |
| C37-H 3 | QVQLVESGGGLVQPGGSLRLSCAASGLTFSDYAIG WFRQAPGKEREGISCIRIYDGNTYYADSVKGRFTISRDN AKNHVYLQMNSLRAEDTAVYYCAAGSYYSCSVYPAYD LDYWGKGTLVTVSS | 6.75 |
| C37-H 4 | QVQLVESGGGLVQPGGSLRLSCAASGLTFSDYAIG WFRQAPGKEREGISCIRIYDGNTYYADSVKGRFTISRDN SKNHVYLQMNSLKPEDTAVYYCAAGSYYSCSVYPAYD LDYWGKGTLVTVSS | 6.75 |

| | | |
|---|---|---|
| Note: The underlined part indicates a mutated amino acid. | | |

After the mutation was completed, the affinity and expression level of each mutant were analyzed, and the specific detection results were shown in Table 3.

**Table 3 Detection of affinity and expression level of humanized variants**

| Designation | Relative activity (%) | Expression level (g/L) | PI value |
|---|---|---|---|
| C37-F1 | 100 | 0.045 | 6.75 |
| C37H | 151 | 0.078 | 6.75 |
| C37-H1 | 110 | 0.016 | 6.75 |
| C37-H2 | 122 | 0.004 | 6.75 |
| C37-H3 | 171 | 0.032 | 6.75 |
| C37-H4 | 141 | 0.052 | 6.75 |

### 1.4 Cystine modification at terminus of C37H antibody

There were a total of four lysines in the sequence of the C37H antibody, and lot-to-lot uncertainty might increase when small molecules such as NOTA/DOTA were conjugated. Therefore, cystine modification was performed on the terminus of the sequence of the C37H antibody for site-directed conjugation. A total of four forms of modification were performed to add a separate cysteine C, as well as three amino acid sequence forms VDC, GGC, and GSC to the terminus, respectively. After the construction was completed, transient transfection was performed, the expression level was calculated, and the conjugation method was optimized and verified separately to select the optimal cystine modification form at the terminus.

Sequences arising from cystine modification at terminus of C37H

| Designation | Sequence | PI value |
|---|---|---|
| C37H-C | QVQLVESGGGLVQPGGSLRLSCAASGLTFSD YAIGWFRQAPGKEREGISCIRIYDGNTYYADSVKG RFTISRDNSKNHVYLQMNSLRAEDTAVYYCAAGS YYSCSVYPAYDLDYWGKGTLVTVSSC | 6.74 |
| C37H-VDC | QVQLVESGGGLVQPGGSLRLSCAASGLTFSD YAIGWFRQAPGKEREGISCIRIYDGNTYYADSVKG RFTISRDNSKNHVYLQMNSLRAEDTAVYYCAAGS YYSCSVYPAYDLDYWGKGTLVTVSSVDC | 5.72 |
| C37H-GSC | QVQLVESGGGLVQPGGSLRLSCAASGLTFSD YAIGWFRQAPGKEREGISCIRIYDGNTYYADSVKG RFTISRDNSKNHVYLQMNSLRAEDTAVYYCAAGS YYSCSVYPAYDLDYWGKGTLVTVSSGSC | 6.74 |
| C37H-GGC | QVQLVESGGGLVQPGGSLRLSCAASGLTFSD YAIGWFRQAPGKEREGISCIRIYDGNTYYADSVKG RFTISRDNSKNHVYLQMNSLRAEDTAVYYCAAGS YYSCSVYPAYDLDYWGKGTLVTVSSGGC | 6.74 |

### Example 2: Precursor Conjugation for NODAGA-GSC-C37H Single-domain Antibody

A bifunctional chelating agent Maleimide-NODAGA was conjugated to the cysteine at the terminus of the C37H-GSC single-domain antibody; a conjugated product was purified through a 10 kDa ultrafiltration tube, and the buffer was changed to pure water; the absorbance was measured by an ultraviolet spectrophotometer at 280 nm; and the conjugation efficiency was analyzed by SEC-HPLC. Its affinity with CD8α protein in vitro was analyzed by ELISA. The method was specifically as follows.

TCEP (Tris(2-carboxyethyl) phosphine hydro-chloride, Sigma) was dissolved in PBS, and the resulting TCEP solution was mixed with C37H-GSC to break a disulfide bond formed by cysteine at the terminus of the protein. After ultrafiltration through the ultrafiltration tube, excess TCEP and free cysteine were removed. The Maleimide-NODAGA solution was mixed with C37H-GSC to react at 37°C. After the reaction, the excess Maleimide-NODAGA was removed with the ultrafiltration tube, giving the product NODAGA-GSC-C37H single-domain antibody.

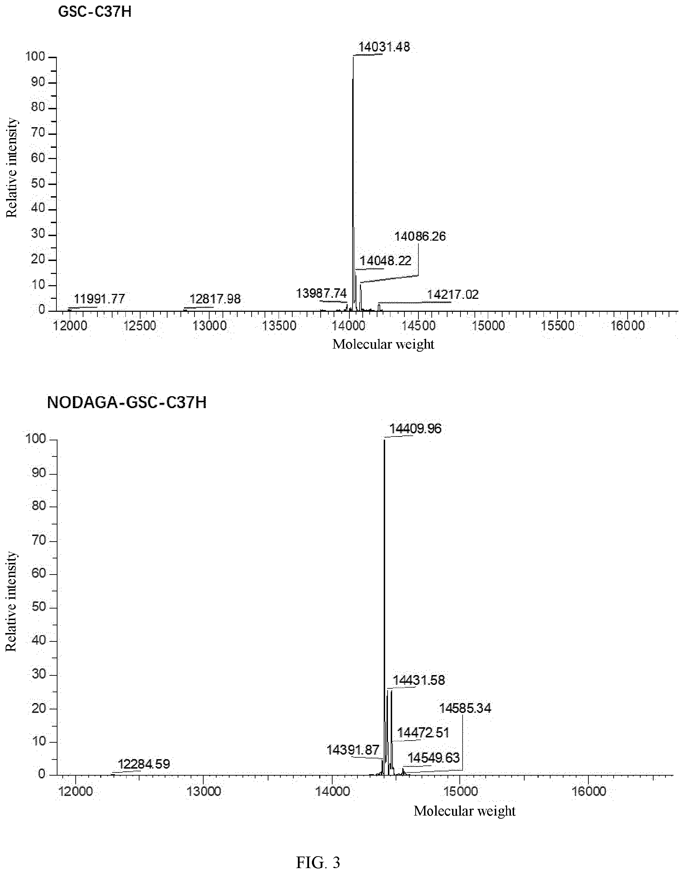

Characterization was performed using SEC-HPLC (FIG. 2) and LC-MS (FIG. 3), and the results showed the molecular weight was slightly increased after the antibody is conjugated with the small molecule, and a peak position was brought forward slightly based on the SEC-HPLC results. The mass spectrometry results showed that the molecular weight of GSC-C37H was 14031, one molecule of cystine (with the molecular weight of 119) was reduced and then conjugated with one molecule of NODAGA (with the molecular weight of 497), and the final molecular weight of NODAGA-GSC-C37H was 14409, which was consistent with the theory.

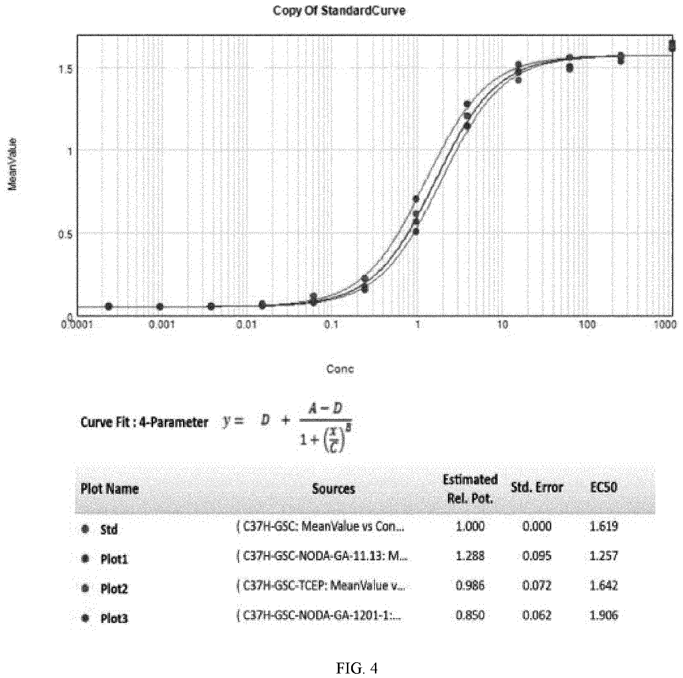

The activity of NODAGA-GSC-C37H after above conjugation was determined by ELISA as follows: a CD8α Fc fusion protein was used to coat a plate at 5 µg/well and let stand overnight at 4°C. After the blocking was performed with 3% BSA at 37°C, serially diluted samples (with an unconjugated C37H-GSC single-domain antibody as a standard) were added to allow action for 1 h at 37°C. Then, anti-his-HRP (available from Abcam) was added to allow reaction for 1 hour at room temperature. Then, a chromogenic solution was added, and the absorption value was read at a wavelength of 450 nm. The ELISA-based activity was summarized in Table 4 and FIG. 4, and the results showed that GSC-C37H was not affected in its capability of binding to an antigen after reduction and after the conjugation with NODAGA.

**Table 4. Summary of ELISA-based activity results of NODAGA-GSC-C37H**

| Single-domain antibody number | Relative activity (%) |
|---|---|
| C37H-GSC | 100 |
| C37H-GSC TCEP after reduction | 96.8 |
| NODAGA-C37H-GSC after conjugation (Lot #1) | 85 |
| NODAGA-C37H-GSC after conjugation (Lot #2) | 128.8 |

### Example 3: Radiolabeling of 68Ga-NODAGA-GSC-C37H

A ⁶⁸Ga eluent was prepared by rinsing an Eckert & Ziegler IGG100 germanium⁶⁸/gallium⁶⁸ (Ge 68/Ga 68) generator with 0.1 M sterile HCl; an equal volume of 0.2 M sodium acetate solution was added; 1/4 volume of 0.1 M sodium acetate buffer containing the above NODAGA-GSC-C37H antibody at pH 5.3 was added, with the pH of the reaction system between 4.5 and 4.7; and the reaction occurred at room temperature for 10 minutes. The unreacted ionic gallium was removed by a PD-10 column, and the resultant was filtered through a membrane of 0.22 um. The product (⁶⁸Ga-NODAGA-GSC-C37H single-domain antibody injection) was subjected to quality control by analysis of pH, Radio-TLC, Radio-HPLC, radioactivity, radiochemical purity or the like. In addition, the ⁶⁸Ga eluent might also be prepared by rinsing the ITG germanium⁶⁸/gallium⁶⁸ (Ge-68/Ga-68) generator with 0.05 M sterile HCl, with other reaction and quality control conditions kept the same.

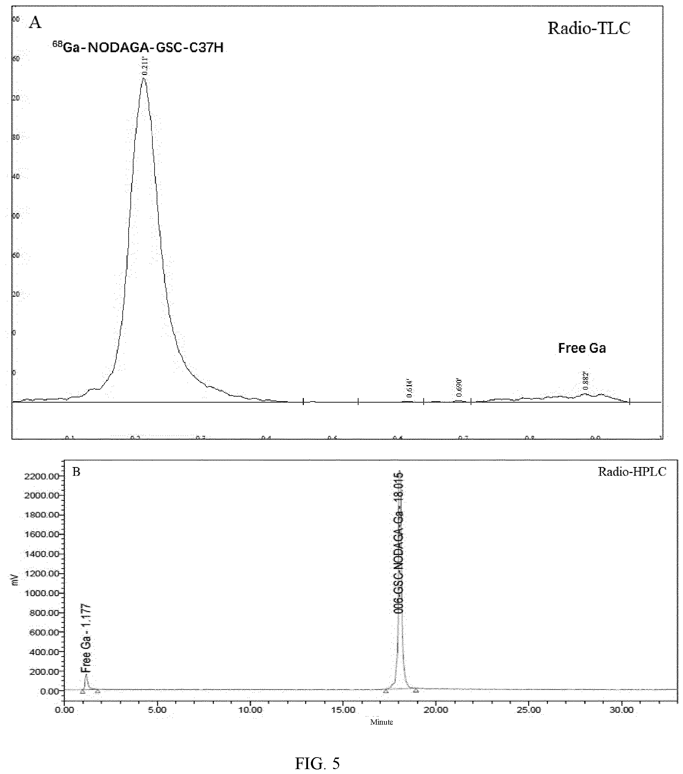

A small number of samples were tested by Radio-TLC and Radio-HPLC, and the radiochemical purity (RCP) was calculated by integrating the peaks on the spectrum. The analysis results were shown in FIGs. 5A-5B, indicating successful labeling of the single-domain antibody by the positron nuclide ⁶⁸Ga. FIG. 5A shows the Radio-TLC results for the radiolabeled sample, and FIG. 5B shows the Radio-HPLC results for the radiolabeled sample.

The Radio-HPLC results are consistent with the Radio-TLC results; the Radio-TLC results show that the free ⁶⁸Ga migrates faster, with a migration factor of about 0.9, while the migration factor of ⁶⁸Ga-NODAGA-GSC-C37H is about 0.2; and the Radio-HPLC results show that the appearance time of the peak of free ⁶⁸Ga is at about the first minute, and the appearance time of the peak of ⁶⁸Ga-NODAGA-GSC-C37H is at about the 18th minute, which is kept substantially the same as that of protein, indicating that the labeling efficiency of both methods is more than 95%.

### Example 4: PET/CT Developing Experiment of HSC-NPG Humanized Animals

### 4.1. HSC-NPG animal models for studies

In vivo studies were performed with 6-8-week-old HSC-NPG mice. For heterotransplantation, 100 µl of MC38-CD8/PBS cells were subcutaneously implanted in the right front leg of each mouse, and meanwhile, 100 µl of MC38/PBS cells were subcutaneously implanted in the left front leg of each mouse. The cell inoculation density was about 5-6×10⁶ cells/mouse. Implantation was performed under isoflurane anesthesia. Under these conditions, usable tumors (100-300 mm3) (MC38-CD8 or MC38) were obtained after 1-2 weeks in more than 90% of the injected animals.

### 4.2. Micro-PET/CT imaging

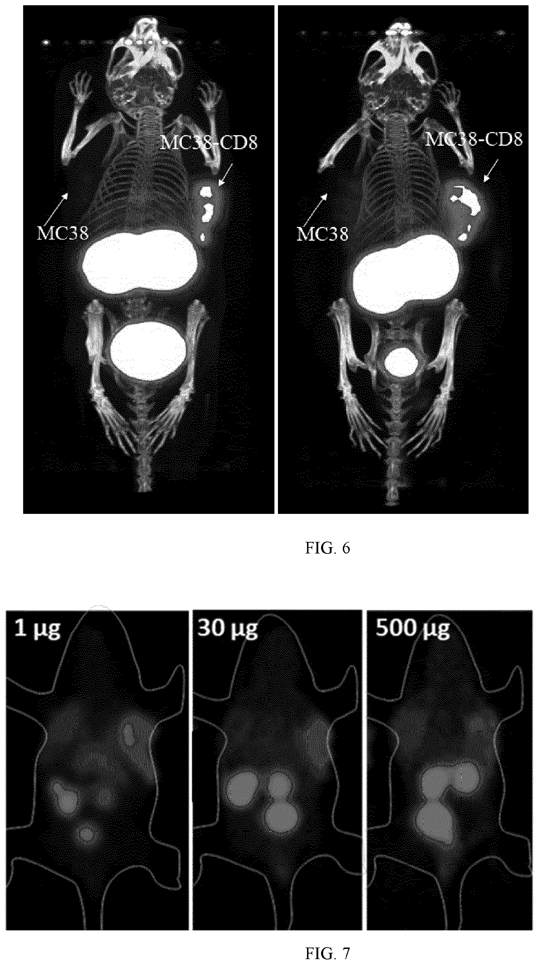

An MC38-CD8+/- tumor model was inoculated according to the method in 4.1. Micro-PET/CT (IRIS PET/CT, inviscan, Strasbourg, France) was used for scanning to measure the %ID/g of lesions. Continuous PET/CT scanning of small animals was performed within 120 minutes to analyze the radioactive absorption in tumors, muscles, heart (blood), liver, spleen, lungs, and kidneys at multiple time points, as shown in FIG. 6.

FIG. 6 shows the PET/CT developing results of HSC-NPG humanized animals, with MC38 transplantation tumors with negatively expressed CD8 on the left and the MC38-CD8 transplantation tumors with positively expressed CD8 on the right.

As shown in FIG. 6, after the injection of the above-mentioned PET tracer ⁶⁸Ga-NODAGA-GSC-C37H, the MC38-CD8 transplantation tumors were clearly visible, while the MC38 transplantation tumors with negatively expressed CD8 did not show uptake after injection. Therefore, the MC38-CD8 tumors showed good discriminability compared with the negative control tumors at the opposite side. The radioactive signals from the kidneys were the highest, indicating that ⁶⁸Ga-NODAGA-GSC-C37H was mainly metabolized via the kidneys.

Compared with non-humanized animals, the HSC-NPG humanized animal model had a certain uptake of ⁶⁸Ga-NODAGA-GSC-C37H in the corresponding liver, spleen, lung and other tissues because the human CD8 was expressed in its heart (blood), liver, spleen, lungs and other tissues. This further demonstrated the CD8 tracing potential of ⁶⁸Ga-NODAGA-GSC-C37H.

### Example 5: Dose Exploration Experiment in PBMC humanized Animals

### 5.1. PBMC animal model for studies.

In vivo studies were performed with 6-8-week-old PBMC mice. For heterotransplantation, 100 µl of MC38-CD8/PBS cells were subcutaneously implanted in the right front leg of each mouse, and meanwhile, 100 µl of MC38/PBS cells were subcutaneously implanted in the left front leg of each mouse. The cell inoculation density was about 5-6×10⁶ cells/mouse. Implantation was performed under isoflurane anesthesia. Under these conditions, usable tumors (100-300 mm3) (MC38-CD8 or MC38) were obtained after 1-2 weeks in more than 90% of the injected animals.

### 5.2. Mode of administration and dose selection

Cold/hot protein co-injection was intended to be used for administering the ⁶⁸Ga-NODAGA-GSC-C37H. In the dose exploration experiment in PBMC humanized animals, a total of 6 dose groups were set up, each with a total protein content of 0.3 µg, 1 µg, 5 µg, 30 µg, 150 µg, or 500 µg; and micro-PET scanning (ROI delineation of tissue distribution), ex vitro tissue distribution, and ex vitro blood collection PK and other experiments were conducted as evaluation methods, to determine the dose-efficacy change relationship in the PBMC humanized animal models, thereby estimating the optimal clinical dose of ⁶⁸Ga-NODAGA-GSC-C37H.

### 5.3. Micro-PET imaging

An MC38-CD8+/- tumor model was inoculated according to the method in 5.1. The mice were anesthetized with isoflurane and placed in PET beds, and the mice were administrated with drugs via tail vein injection in terms of six dose groups (-100 µCi) of 0.3 µg, 1 µg, 5 µg, 30 µg, 150 µg, and 500 µg. Continuous PET scanning was performed within 120 minutes to analyze the radioactive absorption in tumors, heart (blood), liver, spleen, lungs, muscles, kidneys and the like at multiple time points. ROI delineation was performed using MIM software to calculate the biodistribution and the activity-time curves.

FIG. 7 shows the microPET developing images (1 h after dosage) of a dose escalation test in PBMC animal, with MC38 transplantation tumors with negatively expressed CD8 on the left and the MC38-CD8 transplantation tumors with positively expressed CD8 on the right.

As shown by the results in FIG. 7, after the mice were administrated with 0.3-500 µg of radiolabeled single-domain antibody (-100 µCi/0.3-500 µg) via tail vein injection, the MC38-CD8 transplantation tumors were clearly visible, while the MC38 transplantation tumors with negatively expressed CD8 did not show uptake after injection. Therefore, the MC38-CD8 tumors showed good discriminability compared with the negative control tumors at the opposite side. The radioactive signals from the kidneys were the highest, indicating that ⁶⁸Ga-NODAGA-GSC-C37H was mainly metabolized via the kidneys. Compared with non-humanized animals, the PBMC humanized animal model had a certain uptake of ⁶⁸Ga-NODAGA-GSC-C37H in the corresponding liver, spleen, lung and other tissues because the human CD8 was expressed in its heart (blood), liver, spleen, lungs and other tissues.

With the increase of the total protein dose, the uptake by CD8+ tumors decreased gradually to show a significant correlation, while the uptake by CD8+ humanized tissues and organs such as liver, spleen, and lung also decreased accordingly, demonstrating the specific binding of ⁶⁸Ga-NODAGA-GSC-C37H to CD8+.

### 5.4. In vivo distribution

An MC38-CD8+/- tumor model was inoculated according to the method in 5.1. The mice were anesthetized with isoflurane, and then administrated with drugs via tail vein injection in terms of six dose groups (-100 µCi) of 0.3 µg, 1 µg, 5 µg, 30 µg, 150 µg, and 500 µg. Data were acquired after 2 h, and 3 mice were euthanized at each time point. The blood, kidney, liver, spleen, lung, heart, intestine, stomach, muscle, skin, brain, bone, and CD8+/- tumors or other tissues of interest were dissected, and counting was conducted by a γ counter to collect data. The injected dose was the total injected dose. For each organ, the percentage injected dose (%ID) was determined based on this total injected dose, and the organ was weighed to determine the percentage injected dose per gram (%ID/g). After micro-PET developing, the biodistribution results of ROI delineated by the MIM software were compared with the biodistribution results detected by the γ counter ex vivo.

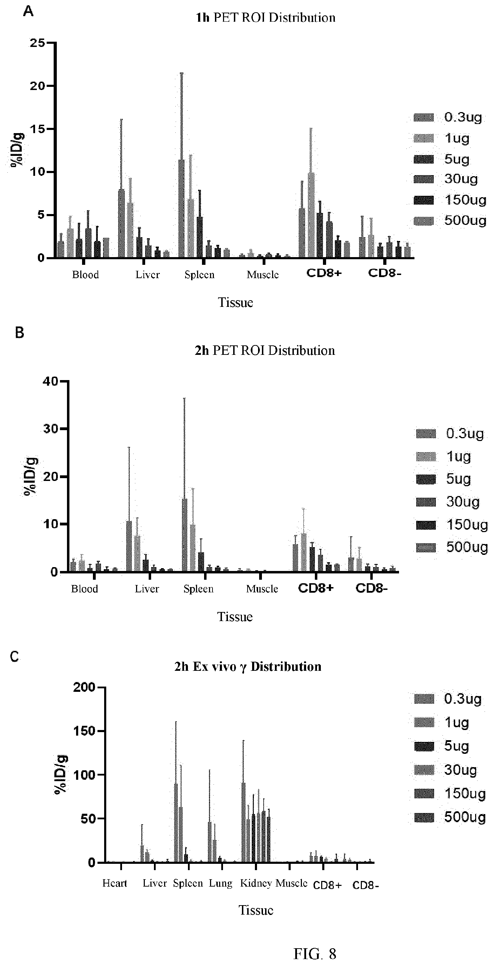

FIG. 8A shows the biodistribution results of ROI delineated by the MIM software after micro-PET developing after 1 h since administration; FIG. 8B shows the biodistribution results of ROI delineated by the MIM software after micro-PET developing after 2 h since administration; and FIG. 8C shows the ex vivo biodistribution results based on dissection after 2 h since administration (n=3).

As shown in FIGs. 8A-8C, the PET scan data after 1 h and 2 h since administration were similar to the results of ex vivo tissue distribution after 2 h. The two detection methods share similar tissue uptake values and its trends over dose. In particular, the uptake by the CD8+ tumor tissues, liver, spleen and lung, and its trends over dose were basically the same.

After the mice were administrated with 0.3-500 µg of radiolabeled single-domain antibody (-100 µCi/0.3-500 µg) via tail vein injection, the MC38-CD8 transplantation tumors showed high uptake, while the MC38 transplantation tumors with negatively expressed CD8 did not show uptake after injection.

With the increase of the total protein dose, the uptake by CD8+ tumors decreased gradually to show a significant correlation, while the uptake by CD8+ humanized tissues and organs such as liver, spleen, and lung also decreased accordingly, demonstrating the specific binding of ⁶⁸Ga-NODAGA-GSC-C37H to CD8+. At the dose of 0.3 µg, the administration dose for the CD8+ positive tumors was not higher than 1 µg; and compared with the dose of 500 µg, the administration dose of 150 µg had completely blocked the uptake by the CD8+-positive tumors. Cross-validation of biodistribution studies of the two detection methods showed that the optimal dose for this animal model was 1 µg and the minimum blocking dose was 150 µg.

### 5.5. Ex vivo pharmacokinetic (PK) in blood collected

An MC38-CD8+/- tumor model was inoculated according to the method in 5.1. The mice were anesthetized with isoflurane, and then administrated with drugs via tail vein injection in terms of six dose groups (-100 µCi) of 0.3 µg, 1 µg, 5 µg, 30 µg, 150 µg, and 500 µg. At 2, 5, 10, 15, 30, 60, 90, and 120 min after administration, 0.5-0.8mL of intraocular canthal blood was collected and weighed, the γ count of blood samples was measured with the γ counter, and the ID%/g was calculated.

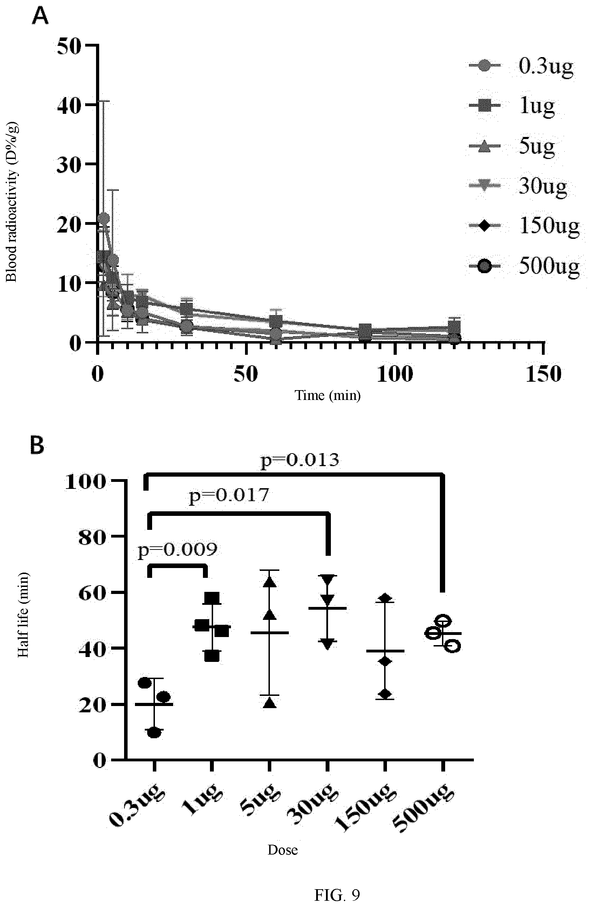

FIG. 9A shows the trend of blood radioactivity ID%/g over time, in which after 0.3-500 µg of radiolabeled single-domain antibody (-100 µCi/0.3-500 µg) was administrated to the mice via tail vein injectione, the blood half-life became shorter and the clearance became faster. These results suggested that ⁶⁸Ga-NODAGA-GSC-C37H had a high metabolic clearance rate like other nanobody-based radioactive drugs. In terms of the general trend, the half-life of radioactivity in the blood was increased and the clearance rate was slowed down as the total protein dose was increased. Moreover, the early uptake was high at a low dose, but with shorter half-life and faster clearance; and the high doses was on the contrary.

FIG. 9B shows the %ID/g TAC curves of radioactive substance uptake in the blood at different doses: the half-life in the low-dose group of 0.3 µg was similar to that of the non-humanized animals at an early stage, which was 20-30 min. However, after the dose was > 1 ug, there was no significant difference in half-life.

### Example 6: Stability Experiment of ⁶⁸Ga-NODAGA-GSC-C37H

### 6.1. In vitro serum stability analysis of ⁶⁸Ga-NODAGA-GSC-C37H

300 µL of human serum was taken; 500 µL of ⁶⁸Ga-NODAGA-GSC-C37H 74 MBq (1 mCi/100 µL PBS) was added; they were mixed and stood at 37°C; and Radio-TLC and Radio-HPLC assays were conducted at 0 h, 0.5 h, 1 h, 2 h, and 4 h.

As shown in FIGs. 10A-10B, there was no significant increase in free 68Ga in serum at 37°C, and no obvious degradation peak was found, indicating stable structure of the radiolabeled antibody, which should have good serum stability in vitro.

### 6.2. In vivo stability analysis of ⁶⁸Ga-NODAGA-GSC-C37H

Normal mice were injected with ⁶⁸Ga-NODAGA-GSC-C37H 74 MBq (1 mCi/100 µL PBS) from their tail veins. After 15 min, 1.5 h and 4 h since injection, urine samples were collected; all samples were dissolved with a certain amount of 50% acetonitrile aqueous solution and centrifuged at 8000 rpm for 15 min; the supernatant was taken and filtered through a filter membrane of 0.22 µm; and the filtrate was analyzed by Radio-TLC and Radio-HPLC.

As shown in FIG. 11A-11B, the content of free ⁶⁸Ga in the urine did not increase significantly after 4 hours since the mice were administered; and the content of free ⁶⁸Ga was not found in the blood samples 30 min after administration, and no other degradation peaks were found increased, indicating that the radiolabeled antibody is substantially present in an intact state in blood and urine with stable structure.

## Claims

1. A CD8α binding polypeptide, comprising at least one variable domain, wherein an amino acid sequence of said variable domain has substitution, deletion or addition of one or more amino acids compared with an amino acid sequence as set forth in SEQ ID NO.: 1.

2. The CD8α binding polypeptide according to claim 1, wherein said CD8α binding polypeptide has one or more of the following properties:
(i) being capable of binding to a packing of a PROTEIN A affinity chromatographic column;
(ii) being capable of binding to CD8α with the same or greater affinity compared with a reference antibody having an amino acid sequence as set forth in SEQ ID NO.: 1;
(iii) an increased expression level compared with the reference antibody having said amino acid sequence as set forth in SEQ ID NO.: 1; and
(iv) an increased isoelectric point compared with the reference antibody having said amino acid sequence as set forth in SEQ ID NO.: 1.

3. The CD8α binding polypeptide according to any one of claims 1-2, comprising an antibody or an antigen-binding fragment thereof.

4. The CD8α binding polypeptide according to claim 3, wherein said antibody comprises a monoclonal antibody, a multispecific antibody, a chimeric antibody, a humanized antibody and/or a fully human antibody.

5. The CD8α binding polypeptide according to any one of claims 3-4, wherein said antigen-binding fragment comprises Fab, Fab', F(ab)2, a Fv fragment, F(ab')2, scFv, di-scFv, VHH and/or dAb.

6. The CD8α binding polypeptide according to claim 5, wherein said VHH is camelid, chimeric, human, partially humanized or fully humanized.

7. The CD8α binding polypeptide according to any one of claims 1-6, wherein said variable domain comprises CDR1, CDR2 and CDR3 in an amino acid sequence as set forth in SEQ ID NO.: 14.

8. The CD8α binding polypeptide according to any one of claims 1-7, wherein said variable domain comprises CDR1, CDR2 and CDR3 in an amino acid sequence as set forth in SEQ ID NO.: 2, SEQ ID NO.: 15 or SEQ ID NO.: 16.

9. The CD8α binding polypeptide according to any one of claims 1-8, wherein said variable domain comprises CDR1, and said CDR1 comprises an amino acid sequence as set forth in SEQ ID NO.: 19.

10. The CD8α binding polypeptide according to any one of claims 1-9, wherein said variable domain comprises CDR1, and said CDR1 comprises an amino acid sequence as set forth in SEQ ID NO.: 17 or SEQ ID NO.: 18.

11. The CD8α binding polypeptide according to any one of claims 1-10, wherein said variable domain comprises CDR2, and said CDR2 comprises an amino acid sequence as set forth in SEQ ID NO.: 23.

12. The CD8α binding polypeptide according to any one of claims 1-11, wherein said variable domain comprises CDR2, and said CDR2 comprises an amino acid sequence as set forth in SEQ ID NO.: 20, SEQ ID NO.: 21 or SEQ ID NO.: 22.

13. The CD8α binding polypeptide according to any one of claims 1-12, wherein said variable domain comprises CDR3, and said CDR3 comprises an amino acid sequence as set forth in SEQ ID NO.: 27.

14. The CD8α binding polypeptide according to any one of claims 1-13, wherein said variable domain comprises CDR3, and said CDR3 comprises an amino acid sequence as set forth in SEQ ID NO.: 24, SEQ ID NO.: 25 or SEQ ID NO.: 26.

15. The CD8α binding polypeptide according to any one of claims 1-14, wherein said variable domain comprises CDR1, CDR2 and CDR3; and said CDR1 comprises an amino acid sequence as set forth in SEQ ID NO.: 19, said CDR2 comprises an amino acid sequence as set forth in SEQ ID NO.: 23, and said CDR3 comprises an amino acid sequence as set forth in SEQ ID NO.: 27.

16. The CD8α binding polypeptide according to any one of claims 1-15, wherein said variable domain comprises CDR1, CDR2 and CDR3; and said CDR1 comprises an amino acid sequence as set forth in SEQ ID NO.: 17, said CDR2 comprises an amino acid sequence as set forth in SEQ ID NO.: 20, and said CDR3 comprises an amino acid sequence as set forth in SEQ ID NO.: 24.

17. The CD8α binding polypeptide according to any one of claims 1-15, wherein said variable domain comprises CDR1, CDR2 and CDR3; and said CDR1 comprises an amino acid sequence as set forth in SEQ ID NO.: 17, said CDR2 comprises an amino acid sequence as set forth in SEQ ID NO.: 20, and said CDR3 comprises an amino acid sequence as set forth in SEQ ID NO.: 28.

18. The CD8α binding polypeptide according to any one of claims 1-15, wherein said variable domain comprises CDR1, CDR2 and CDR3; and said CDR1 comprises an amino acid sequence as set forth in SEQ ID NO.: 18, said CDR2 comprises an amino acid sequence as set forth in SEQ ID NO.: 23, and said CDR3 comprises an amino acid sequence as set forth in SEQ ID NO.: 26.

19. The CD8α binding polypeptide according to any one of claims 1-18, wherein compared with the reference antibody having said amino acid sequence as set forth in SEQ ID NO.: 1, said variable domain comprises amino acid substitution(s) at one or more positions selected from the group consisting of:
(a) E30; (b) S60; (c) E62; (d) S72; (e) Q78; (f) I83; (g) T85; (h) D109; and (i) M112.

20. The CD8α binding polypeptide according to any one of claims 1-19, wherein compared with the reference antibody having said amino acid sequence as set forth in SEQ ID NO.: 1, said variable domain comprises one or more amino acid substitutions selected from the group consisting of:
(a) E30S, E30G, E30T or E30Y; (b) S60Y; (c) E62D; (d) S72R, S72H or S72K; (e) Q78H or Q78T; (f) I83M; (g) T85S; (h) D109A, D109V, D109L, D109I, D109G, D109S or D109T; and (i) M112L, M112A, M112V, M112I, M112G, M112S or M112L.

21. The CD8α binding polypeptide according to any one of claims 1-20, wherein compared with the reference antibody having said amino acid sequence as set forth in SEQ ID NO.: 1, said variable domain comprises amino acid substitution(s) selected from one or more of the following groups:
(i) an amino acid substitution of S60Y/E62D/Q78H/I83M/T85S;
(ii) an amino acid substitution of S72R, T85S, D109A, S72R/D109A or S72R/T85S/D109A; and
(iii) an amino acid substitution of E30S/M112L.

22. The CD8α binding polypeptide according to any one of claims 1-21, wherein said variable domain is subject to humanized modification.

23. The CD8α binding polypeptide according to any one of claims 1-22, wherein compared with the reference antibody having said amino acid sequence as set forth in SEQ ID NO.: 1, said variable domain comprises amino acid substitution(s) at one or more positions selected from the group consisting of:
(a) L2; (b) A14; (c) G56; (d) N57; (e) A75; (f) K87; (g) P88; and (h) K117.

24. The CD8α binding polypeptide according to any one of claims 1-23, wherein compared with the reference antibody having said amino acid sequence as set forth in SEQ ID NO.: 1, said variable domain comprises one or more amino acid substitutions selected from the group consisting of:
(a) L2V; (b) A14P; (c) G56R; (d) N57R; (e) A75S; (f) K87R; (g) P88A; and (h) K117Q.

25. The CD8α binding polypeptide according to any one of claims 1-24, wherein compared with the reference antibody having said amino acid sequence as set forth in SEQ ID NO.: 1, said variable domain comprises the following amino acid substitutions: L2Y and A14P.

26. The CD8α binding polypeptide according to any one of claims 1-25, wherein compared with the reference antibody having said amino acid sequence as set forth in SEQ ID NO.: 1, said variable domain further comprises the following amino acid substitutions: A75S, K87R, P88A and/or K117Q.

27. The CD8α binding polypeptide according to any one of claims 1-26, wherein compared with the reference antibody having said amino acid sequence as set forth in SEQ ID NO.: 1, said variable domain further comprises the following amino acid substitutions: G56R and/or N57R.

28. The CD8α binding polypeptide according to any one of claims 1-27, wherein said variable domain comprises an amino acid sequence as set forth in SEQ ID NO.: 14.

29. The CD8α binding polypeptide according to any one of claims 1-28, wherein said variable domain comprises an amino acid sequence as set forth in SEQ ID NO.: 2, SEQ ID NO.: 15 or SEQ ID NO.: 16.

30. The CD8α binding polypeptide according to any one of claims 1-29, wherein said variable domain comprises an amino acid sequence as set forth in SEQ ID NO.: 3, SEQ ID NO.: 4, SEQ ID NO.: 5, SEQ ID NO.: 6, SEQ ID NO.: 7, SEQ ID NO.: 8, SEQ ID NO.: 9, SEQ ID NO.: 10, SEQ ID NO.: 11, SEQ ID NO.: 12 or SEQ ID NO.: 13.

31. The CD8α binding polypeptide according to any one of claims 1-30, wherein a C-terminus of said CD8α binding polypeptide further comprises a cysteine modification.

32. The CD8α binding polypeptide according to claim 31, wherein said cysteine modification comprises the following forms: C, VDC, (GG)ₙC or (GS)ₘC, with n or m each independently selected from the group consisting of 1, 2, 3 and 4.

33. The CD8α binding polypeptide according to any one of claims 1-32, wherein said CD8α binding polypeptide comprises one variable domain.

34. The CD8α binding polypeptide according to any one of claims 1-33, wherein said CD8α binding polypeptide comprises an amino acid sequence as set forth in SEQ ID NO.: 29, SEQ ID NO.: 21 or SEQ ID NO.: 32.

35. An isolated nucleic acid molecule or isolated nucleic acid molecules, encoding the CD8α binding polypeptide of any one of claims 1-34.

36. A vector, comprising said nucleic acid molecule or nucleic acid molecules according to claim 35.

37. A cell, comprising the nucleic acid molecule or nucleic acid molecules of claim 35 or the vector of claim 36.

38. A method for preparing the CD8α binding polypeptide of any one of claims 1-34, comprising culturing the cell of claim 37 under a condition allowing expression of said CD8α binding polypeptide.

39. The method according to claim 38, further comprising recovering a CD8α binding polypeptide expressed by said cell.

40. The method according to claim 39, further comprising purifying and/or modifying said CD8α binding polypeptide.

41. An immunoconjugate, comprising the CD8α binding polypeptide of any one of claims 1-34.

42. The immunoconjugate according to claim 41, further comprising at least one detectable marker binding to said CD8α binding polypeptide.

43. The immunoconjugate according to claim 42, wherein said detectable marker is selected from the group consisting of a radionuclide, a fluorescent agent, a chemiluminescent agent, a bioluminescent agent, a paramagnetic ion, and an enzyme.

44. The immunoconjugate according to claim 42, wherein said detectable marker comprises ¹¹⁰In, ¹¹¹In, ¹⁷⁷Lu, ¹⁸F, ⁵²Fe, ⁶²Cu, ⁶⁷Cu, ⁶⁷Ga, ⁶⁸Ga, ⁶⁸Ge, ⁸⁶Y, ⁹⁰Y, ⁸⁹Zr, ^{94m}Tc, ¹²⁰I, ¹²³I, ¹²⁴I, ¹²⁵I, ¹³¹I, ¹⁵⁴⁻¹⁵⁸Gd, ³²P, ¹¹C, ¹³N, ¹⁵O, ¹⁸⁶Re, ¹⁸⁸Re, ⁵¹Mn, ^{52m}Mn, ⁷²As, ⁷⁵Br, ⁷⁶Br, ^{82m}Rb, ⁸³Sr or other γ-, β-, or positron emitters.

45. The immunoconjugate according to claim 42, wherein said CD8α binding polypeptide is conjugated with said detectable marker via a chelating agent.

46. The immunoconjugate according to claim 42, wherein said chelating agent is selected from the group consisting of DTPA, EDTA, NOTA, DOTA, TRAP, TETA, NETA, CB-TE2A, Cyclen, Cyclam, Bispidine, TACN, ATSM, SarAr, AmBaSar, MAG3, MAG2, HYNIC, DADT, EC, NS3, H2dedpa, HBED, DFO, PEPA or HEHA, and their derivatives.

47. The immunoconjugate according to any one of claims 42-46, wherein said detectable marker comprises ⁶⁸Ga.

48. The immunoconjugate according to any one of claims 45-47, wherein said chelating agent comprises NOTA or a derivative thereof.

49. The immunoconjugate according to claims 48, wherein said chelating agent comprises NODAGA or Maleimide-NODAGA.

50. A composition, comprising the CD8α binding polypeptide of any one of claims 1-34, the nucleic acid molecule or nucleic acid molecules of claim 35, the vector of claim 36, the cell of claim 37 and/or the immunoconjugate of any one of claims 41- 49, and optionally, a pharmaceutically acceptable carrier.

51. The composition according to claim 50, comprising the CD8α binding polypeptide of any one of claims 1-34 or the immunoconjugate of any one of claims 41- 49, wherein said composition is a detecting agent.

52. The composition according to claim 51, wherein said detecting agent is a contrast agent.

53. The composition according to claim 52, wherein said contrast agent is an ECT contrast agent.

54. The composition according to claim 53, wherein said ECT contrast agent comprises an SPECT contrast agent or a PET contrast agent.

55. Use of the CD8α binding polypeptide of any one of claims 1-34, the nucleic acid molecule or nucleic acid molecules of claim 35, the vector of claim 36, the cell of claim 37, the immunoconjugate of any one of claims 41- 49, and/or the composition of any one of claims 50-54 in preparation of a medicament for monitoring, preventing, alleviating and/or treating tumors.

56. Use of the CD8α binding polypeptide of any one of claims 1-34 or the immunoconjugate of any one of claims 41- 49 in preparation of a detecting agent for detecting CD8-positive cells.

57. A method for detecting presence and/or amount of CD8 in a biological sample, comprising contacting said biological sample with the CD8α binding polypeptide of any one of claims 1-34, the immunoconjugate of any one of claims 41- 49, and the composition of any one of claims 50-54.

58. The method according to claim 57, wherein said contacting is conducted in vitro or ex vivo.

59. The method according to any one of claims 57-58, wherein said biological sample is a tissue.

60. The method according to claim 59, wherein said tissue is selected from the group consisting of a blood tissue, a lymphoid tissue and a tumor tissue.

61. The method according to any one of claims 57-60, comprising detecting presence and/or amount of CD8-positive cells in said biological sample.

62. The method according to claim 61, wherein said presence and/or amount of said CD8-positive cells in said biological sample is determined by imaging.

63. The method according to claim 62, wherein said presence and/or amount of said CD8-positive cells in said biological sample is determined by flow cytometry.

64. The method according to any one of claims 61-63, wherein said CD8-positive cells are CD8-positive T cells.

65. A method for detecting and/or diagnosing CD8-related diseases or disorders, comprising administering the CD8α binding polypeptide of any one of claims 1-34, the immunoconjugate of any one of claims 41-49 or the composition of any one of claims 50-54 to a subject in need thereof.

66. The method according to claim 65, further comprising performing imaging on said subj ect.

67. The method according to claim 66, wherein said imaging comprises ECT imaging.

68. The method according to claim 67, wherein said ECT imaging comprises SPECT imaging or PET imaging.

69. The method according to any one of claims 65-68, wherein said CD8-related diseases or disorders comprise tumors.

70. A method for treating tumors, comprising:
1) administering the CD8α binding polypeptide of any one of claims 1-34, the immunoconjugate of any one of claims 41-49 or the composition of any one of claims 50-54 to a subject in need thereof; and
2) determining whether a tumor in said subject comprises CD8-positive cells,
wherein if presence of said CD8-positive cells in said tumor is detected, an anti-tumor therapy is administered to said subject.

71. A method for monitoring efficacy of an anti-tumor therapy in a subject, comprising:
1) administering the CD8α binding peptide of any one of claims 1-34, the immunoconjugate of any one of claims 41-49, or the composition of any one of claims 50-54 to said subject suffering from a tumor and treated with said anti-tumor therapy; and
2) determining an amount of CD8-positive cells in a tumor of said subject.

72. The method according to any one of claims 70-71, wherein said presence and/or amount of said CD8-positive cells in said tumor of said subject is determined by imaging.

73. The method according to any one of claims 70-72, wherein said anti-tumor therapy is an immune checkpoint inhibitor therapy.

74. The method according to any one of claims 70-73, wherein said anti-tumor therapy is selected from administration of a PD-1 inhibitor, a PD-L1 inhibitor, a CTLA-4 inhibitor, a TIM3 inhibitor, a BTLA inhibitor, a TIGIT inhibitor, a CD47 inhibitor, a GITR inhibitor, an LAG3 inhibitor, an additional T cell co-inhibitor or a ligand antagonist, an indolamine-2,3-dioxygenase IDO inhibitor, a vascular endothelial growth factor VEGF antagonist, an Ang2 inhibitor, a transforming growth factor β TGFβ inhibitor, an epidermal growth factor receptor EGFR inhibitor, a CD20 inhibitor, an antibody or a vaccine for a tumor-specific antigen, an adjuvant to increase antigen presentation, a bispecific antibody, a cytotoxin, a chemoagent, cyclophosphamide, a radiotherapy, an IL-6R inhibitor, an IL-4R inhibitor, an IL-10 inhibitor, a cytokine, and an antibody-drug conjugate ADC.

75. The method according to any one of claims 70-74, wherein said tumor comprises a solid tumor.

76. The method according to claim 75, wherein said solid tumor is selected from the group consisting of a colorectal cancer, an ovarian cancer, a prostate cancer, a breast cancer, a brain cancer, a cervical cancer, a bladder cancer, an anal cancer, an uterine cancer, a colon cancer, a liver cancer, a pancreatic cancer, a lung cancer, an endometrial cancer, a bone cancer, a testicular cancer, a skin cancer, a renal cancer, a gastric cancer, an esophageal cancer, a head and neck cancer, a salivary gland cancer and myeloma.

77. A method for isolating CD8-positive cells, comprising: contacting a cell population comprising CD8-positive cells with the CD8α binding polypeptide of any one of claims 1-34 or the immunoconjugate of any one of claims 41-49, and recovering CD8-positive cells binding to the CD8α binding polypeptide of any one of claims 1-34 or the immunoconjugate of any one of claims 41-49.

78. The method according to claim 77, wherein said CD8-positive cells are CD8-positive T cells.

79. The method according to claim 78, wherein said cell population comprising CD8-positive cells is human peripheral blood mononuclear cells PBMCs.

80. The method according to any one of claims 77-79, wherein the CD8α binding polypeptide of any one of claims 1-34 or the immunoconjugate of any one of claims 41-49 is immobilized on a surface of a solid.

81. The method according to claim 80, wherein said solid comprises a gel or a magnetic bead.

82. A kit, comprising the CD8α binding polypeptide of any one of claims 1-34, the immunoconjugate of any one of claims 41-49 or the composition of any one of claims 49-54.
